# EUROPEAN PATENT APPLICATION

(11) **EP 4 819 146 A1**
(43) Date of publication of application: **07.10.2026**
(21) Application number: 26158489.0
(22) Date of filing: 13.02.2026
(51) Int. Cl.: G16B 25/10, G16B 40/20, G16H 50/20, G16H 50/30

(54) **STAINING KIT AND METHOD FOR ASSESSING LIKELIHOOD OF RHEUMATOID ARTHRITIS DISEASE RISK USING THE SAME**

(30) Priority: 31.03.2025 US 202519096053
(71) Applicant: Fullhope Biomedical Co., Ltd., New Taipei City 24159 (TW)
(72) Inventor: Lee, Jan-Mou, 241 New Taipei City (TW); Fang, Chih-Hao, 241 New Taipei City (TW); Li, Po-Yu, 241 New Taipei City (TW); Chan, Chun-Wen, 241 New Taipei City (TW); Chu, Yen-Ying, 241 New Taipei City (TW); Lee, Cheng-Yun, 241 New Taipei City (TW); Chen, Yu-Hua, 241 New Taipei City (TW)
(74) Representative: Engelhard, Markus

(57) **Abstract**

A staining kit is provided, including a first pattern including antibodies against CD3, CD4, CD8, CD14, CD19, CD25, CD45, CD56, CTLA4, FoxP3 and PD-1; a second pattern including antibodies against CD3, CD4, CD14, CD19, CD45, CD56, HLA-DR, PD-1 and PD-L1; a third pattern including antibodies against CD3, CD11b, CD14, CD16, CD19, CD45, CD56, CD66b, CD123 and CD193; and a fourth pattern including antibodies against CD10, CD19, CD21, CD23, CD38, CD45, CD127, IgG and IgM, wherein the antibodies of each pattern are labeled with fluorescent dyes. A method of assessing the likelihood of a rheumatoid arthritis (RA) disease risk in a subject using the staining kit is also provided.

## Description

### FIELD OF INVENTION

The present disclosure relates to a staining kit and uses thereof, and in particular to a method for assessing the likelihood of a rheumatoid arthritis (RA) disease risk in a subject using the staining kit.

### BACKGROUND OF THE INVENTION

A rheumatoid arthritis (RA) disease is an autoimmune disorder that primarily manifests as chronic inflammatory arthritis affecting multiple joints. In 2020, the prevalence of RA disease was 208.8 cases per 100,000 population, with annual increases observed since 1990. It is projected to affect 31.7 million patients by 2050. Although a global investigation suggests an annual decrease in RA-associated mortality, systemic complications arising from uncontrolled disease progression increase years lived with disability and lead to higher socioeconomic costs. Therefore, accurate diagnosis and prompt treatment are essential to mitigate the growing disability burden associated with RA disease.

The diagnosis of RA disease can follow the diagnostic algorithm jointly developed by the American College of Rheumatology (ACR) and the European Alliance of Associations for Rheumatology (EULAR), which evaluates the number of affected joints and the presence of anti-citrullinated protein antibodies (ACPA) or rheumatoid factor (RF). However, the heterogeneous symptoms of RA disease limit the sensitivity (73.5%) and specificity (60%) of this algorithm. Additionally, confirmation of RA disease relies on the seropositivity of ACPA or RF, while at least 30% of RA patients are seronegative for either ACPA or RF, which can delay treatment. This highlights that achieving a precise diagnosis of RA disease remains a significant challenge.

The treatment of RA disease typically involves the use of glucocorticoids, conventional synthetic disease-modifying antirheumatic drugs (DMARDs), and biological DMARDs (such as anti-tumor necrosis factor-α, anti-B-cell and anti-T-cell therapies) to attenuate inflammatory responses and modulate disease activity. However, 6% to 21% of RA patients fail to respond to DMARDs. These difficult-to-treat patients have limited treatment options, highlighting an urgent unmet medical need for better disease control in this population. Furthermore, the efficacy of these treatments is assessed using various scoring tools, such as the disease activity score (DAS) and the patient-derived clinical disease activity index (CDAI). These tools rely on macroscopic observation of joint lesions and changes in serum inflammatory markers, such as the erythrocyte sediment rate (ESR) and serum C-reactive protein (CRP) levels. However, they do not provide real-time insights into immune system changes induced by treatment, making it challenging for physicians to adjust regimens promptly to optimize efficacy. Therefore, there is a critical need not only for improved treatment options for RA patients but also for real-time monitoring tools to track the immune response dynamics during treatment.

Since immune dysregulation plays a key role in the pathogenesis of RA disease, understanding changes in the immune system can help physicians monitor disease activity in real time and adjust therapeutic strategies with greater precision. Recent studies examining the immune cell profile (ICP) in RA patients typically focus on the peripheral and synovial profiles of specific immune cell lineages, such as B cells or CD4+ T cells, and cytokines during disease progression. However, these studies lack a comprehensive evaluation of the immune system as a whole. Consequently, changes in the peripheral ICP remain poorly understood.

### SUMMARY OF THE INVENTION

In view of the description above, the present invention aims to comprehensively compare immune cell subsets between patients having a rheumatoid arthritis (RA) disease and healthy controls to determine the characterized immune cell subsets of the RA disease used for assessing the likelihood of a RA disease risk in a subject.

In one aspect, a method processing biomedical data to generate a probability-based risk indicator for assessing the likelihood of a RA disease risk in a subject is provided, including steps of:
(a) staining peripheral blood mononuclear cells (PBMCs) and/or white blood cells (WBCs) from the subject by using a staining kit, wherein the staining kit includes:
   a first pattern, including antibodies against CD3, CD4, CD8, CD14, CD19, CD25, CD45, CD56, CTLA4, FoxP3 and PD-1;
   a second pattern, including antibodies against CD3, CD4, CD14, CD19, CD45, CD56, HLA-DR, PD-1 and PD-L1;
   a third pattern, including antibodies against CD3, CD11b, CD14, CD16, CD19, CD45, CD56, CD66b, CD123 and CD193; and
   a fourth pattern, including antibodies against CD10, CD19, CD21, CD23, CD38, CD45, CD127, IgG and IgM,
   wherein the antibodies of each pattern are labeled with fluorescent dyes;
(b) performing biomedical data acquisition of fluorescent intensity of each antibody bound to characterized immune cell subsets of an RA disease by using flow cytometry to obtain a dataset including data related to types of the characterized immune cell subsets and proportions thereof; and
(c) evaluating the dataset by using an artificial intelligent (AI) model to generate the probability-based risk indicator for the subject, thereby stratifying the subject into a risk category for the RA disease, wherein the generated probability-based risk indicator reflects the likelihood of the subject being at risk for the RA disease, providing a basis for further clinical evaluation or monitoring.

In another aspect, the present disclosure provides a staining kit. The staining kit includes a first pattern including antibodies against CD3, CD4, CD8, CD14, CD19, CD25, CD45, CD56, CTLA4, FoxP3 and PD-1; a second pattern including antibodies against CD3, CD4, CD14, CD19, CD45, CD56, HLA-DR, PD-1 and PD-L1; a third pattern including antibodies against CD3, CD11b, CD14, CD16, CD19, CD45, CD56, CD66b, CD123 and CD193; and a fourth pattern including antibodies against CD10, CD19, CD21, CD23, CD38, CD45, CD127, IgG and IgM, wherein the antibodies of each pattern are labeled with fluorescent dyes.

Therefore, the present disclosure at least provides the following advantages:
1. The present disclosure provides a novel method for assessing the likelihood of the RA disease risk by combining a staining kit with a flow cytometry and an AI model trained through the analyses of a Boruta algorithm and a machine learning algorithm based on LR algorithm, thereby constructing a potent tool for supporting further clinical evaluation or decision-making.
2. The performance of the claimed invention can achieve a high AUC (*e.g.,* up to 1.0000), sensitivity (*e.g.,* up to 100%) and specificity (*e.g.,* up to 100%) through LR algorithm analyses.
3. The staining kit of the claimed invention can be applied to easily and rapidly identify the characterized immune cell subsets of RA disease.
4. The staining kit of the claimed invention can be utilized to efficiently and accurately assess the likelihood of the RA risk in subjects.

### BRIEF DESCRIPTION OF THE DRAWINGS

The present disclosure file contains at least one drawing executed in color. Copies of this patent or patent application publication with color drawing(s) will be provided by the Office upon request and payment of the necessary fee.

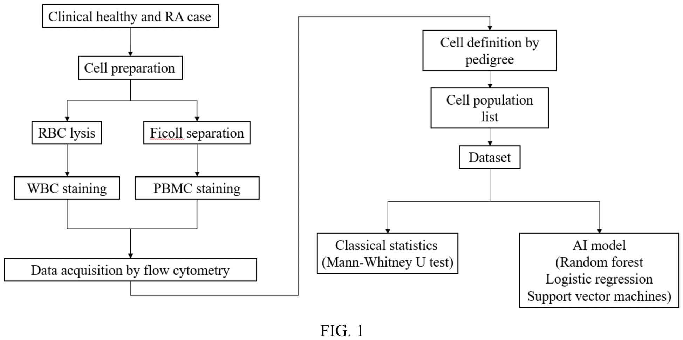

**FIG. 1** illustrates a flow chart of determining the characterized immune cell subsets of RA.

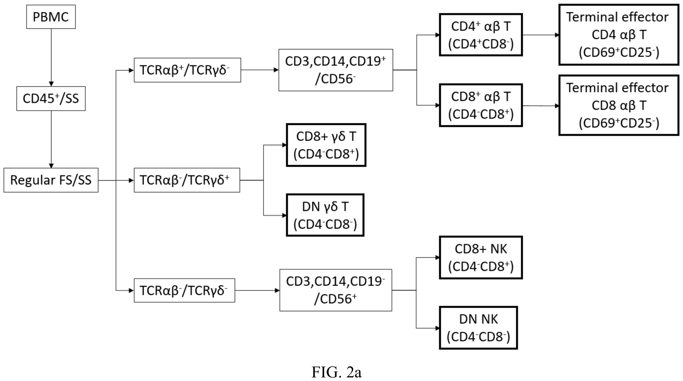

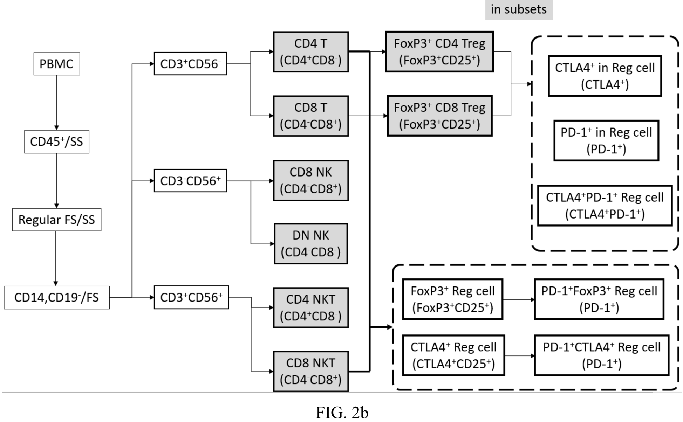

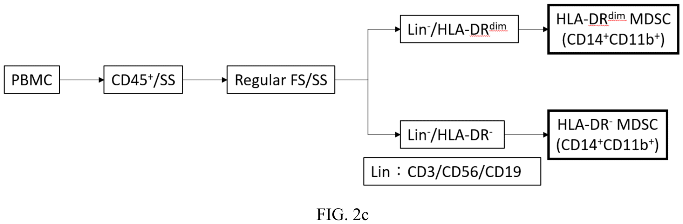

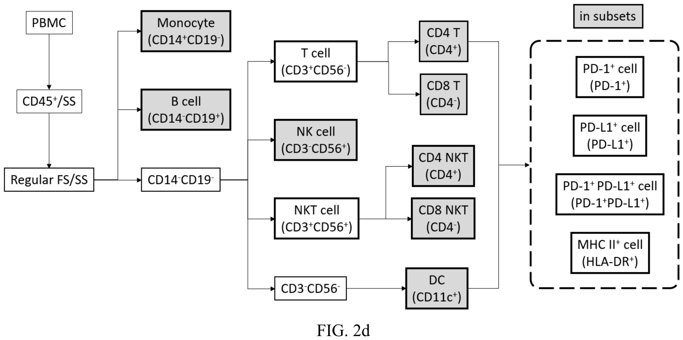

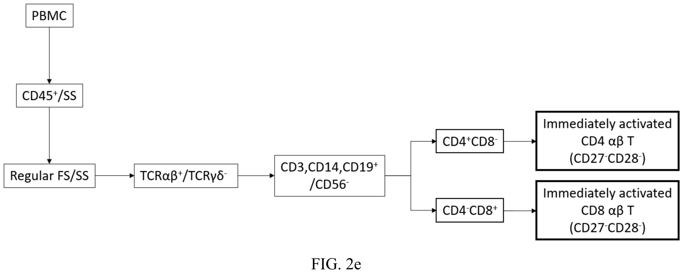

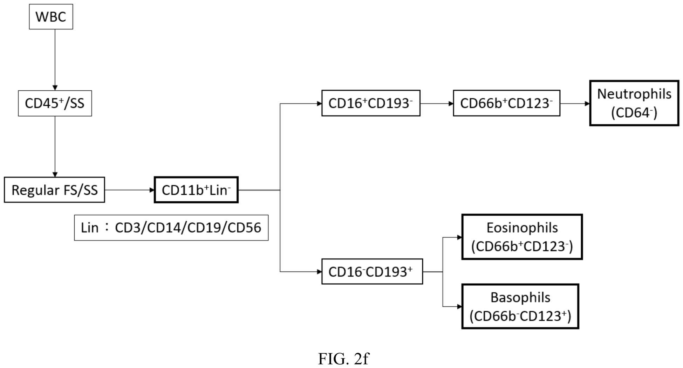

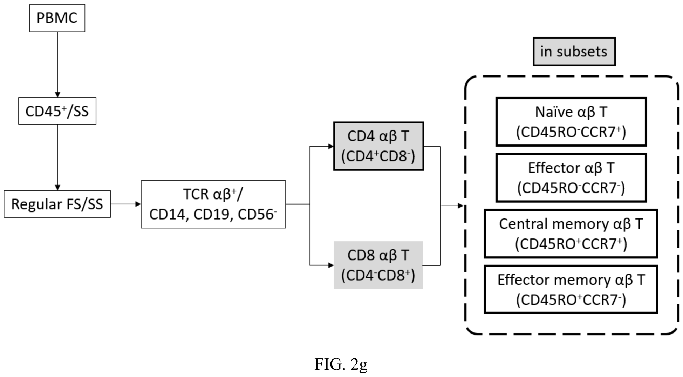

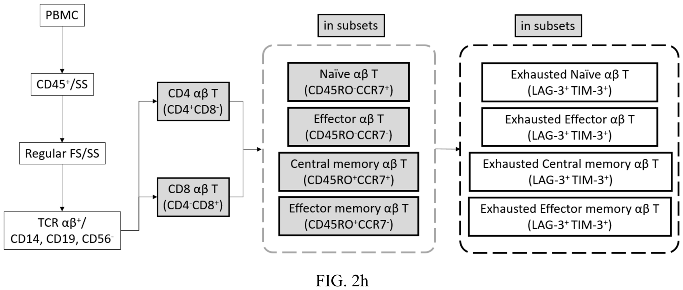

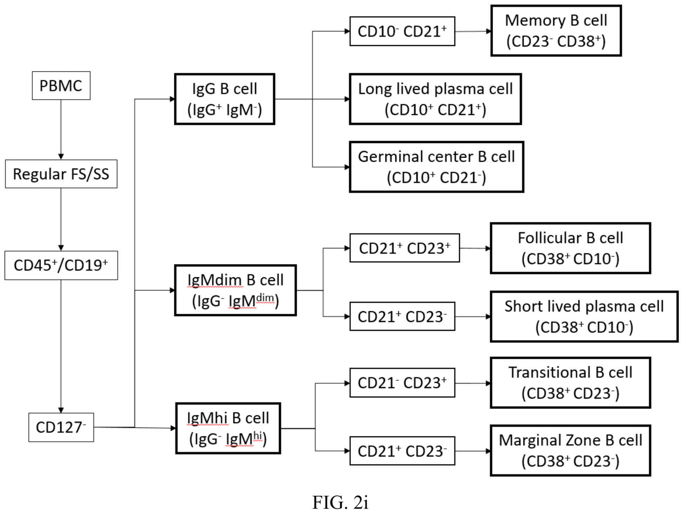

**FIGs. 2a-2i** illustrate the filtrating processes for obtaining immune cell subsets using a pedigree method, wherein **FIG. 2a** illustrate the pattern PT-64 pedigree; **FIG. 2b** illustrates the pattern PT-65 pedigree; **FIG. 2c** illustrates the pattern PT-66 pedigree; **FIG. 2d** illustrates the pattern PT-67 pedigree; **FIG. 2e** illustrates the pattern PT-68 pedigree; **FIG. 2f** illustrates the pattern PT-83 pedigree; **FIG. 2g** illustrates the pattern PT-85 pedigree; **FIG. 2h** illustrates the pattern PT-86 pedigree; and **FIG. 2i** illustrates the pattern PT-87 pedigree.

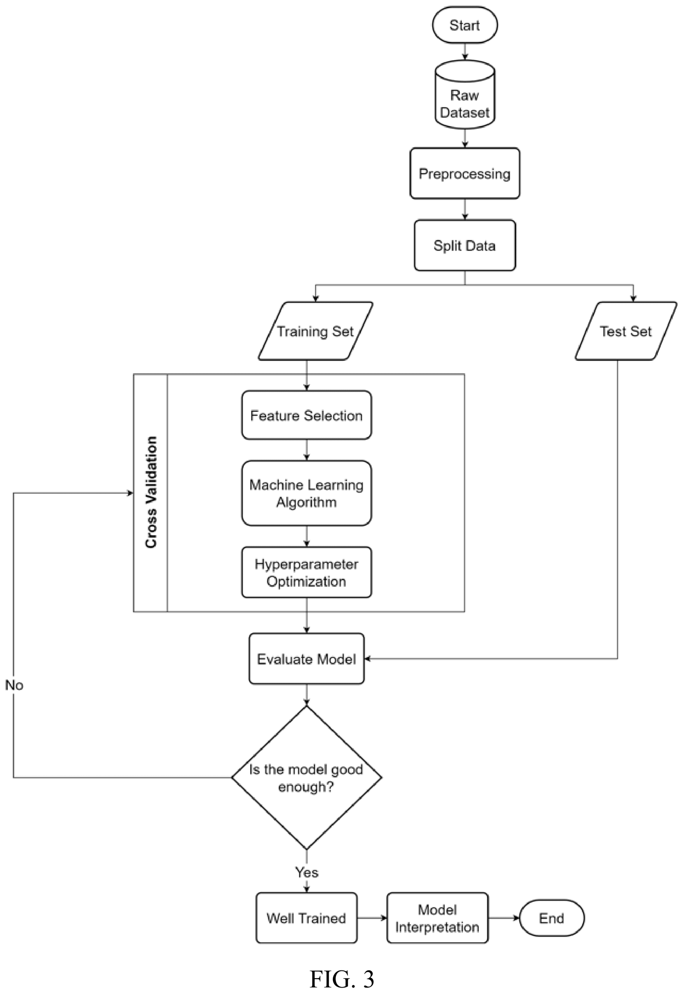

**FIG. 3** illustrates a flow chart of an AI model training developed using Python and trained with a machine learning algorithm using scikit-learn.

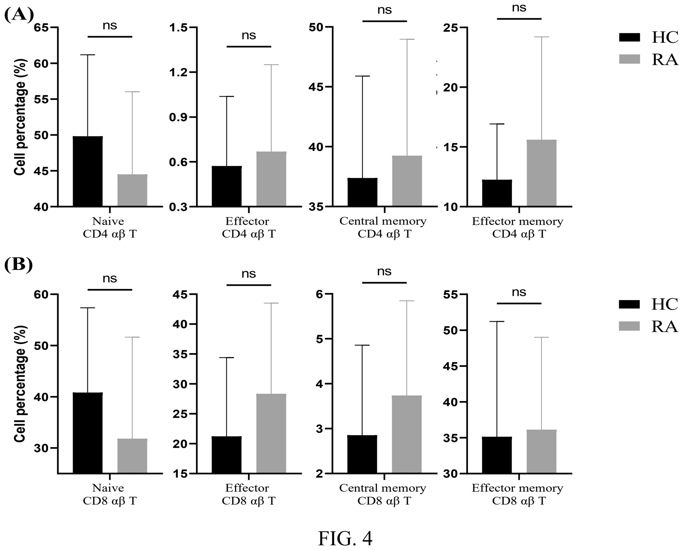

**FIG. 4** illustrates RA patients exhibited activated trend in cellular immunity, wherein the immune cell subsets are presented belonging to **(A)** CD4 T and **(B)** CD8 T cells and compared their proportion between the RA and HC groups, and the results of the comparisons were presented in bar charts.

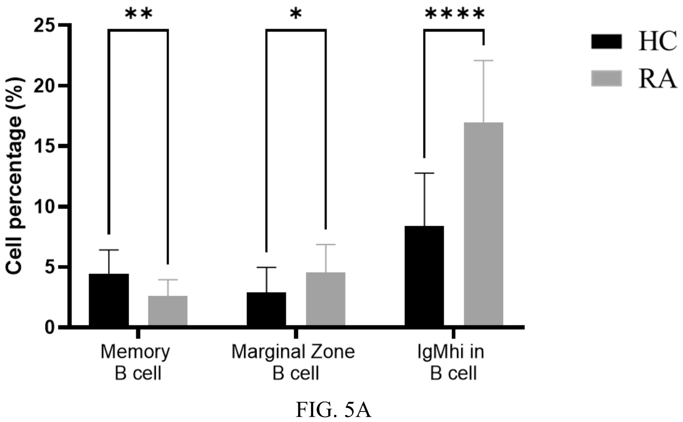

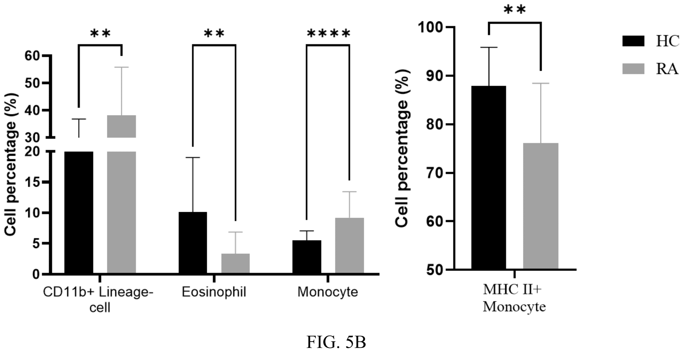

**FIGs. 5A** **and** **5B** illustrate patients with rheumatoid arthritis (RA) exhibited activated humoral immunity and innate immunity, wherein the immune cell subsets belonging to **(****FIG. 5A****)** B-cell lineage and **(****FIG. 5B****)** innate immunity which showed statistically different proportions between the patients with RA (the RA group) and healthy controls (the health group), and the results of these comparisons are presented in a bar chart with * *(p* < 0.05), ** *(p* < 0.01), and **** *(p <* 0.0001) labeling.

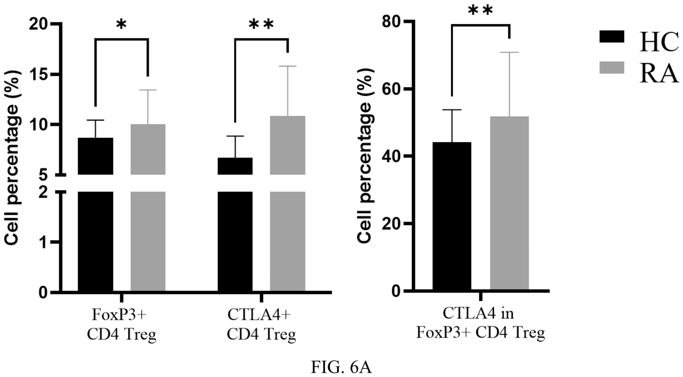

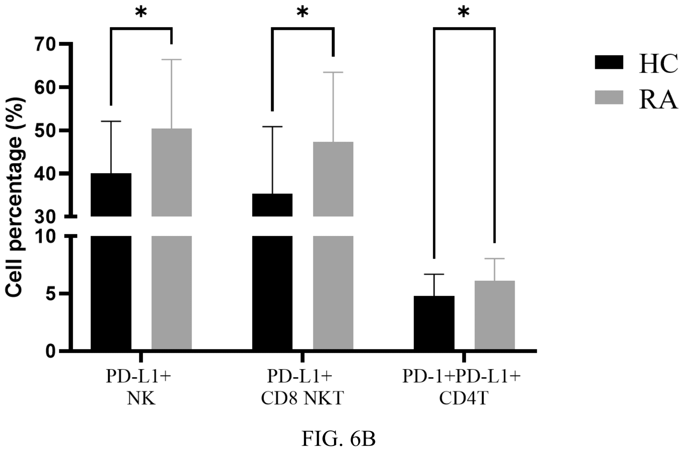

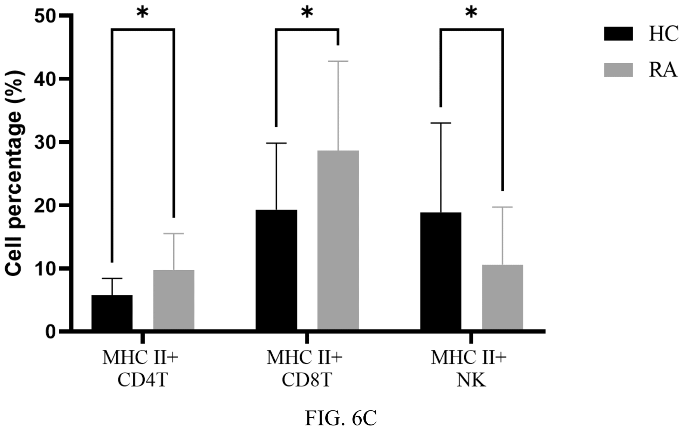

**FIGs. 6A-6C** illustrate immune cell subsets belonging to immune tolerance were compensatively augmented in the patients with RA, wherein the ICP related to immune tolerance and presented the immune cell subsets with statistically difference in proportions between the RA and the health groups; those immune cell subsets were included in **(****FIG. 6A****)** regulatory, **(****FIG. 6B****)** PD-1+/PD-L1+, and **(****FIG. 6C****)** MHC II+ T cells, and the results of these comparisons are displayed in a bar chart with * *(p* < 0.05) and ** (*p* < 0.01) indicating.

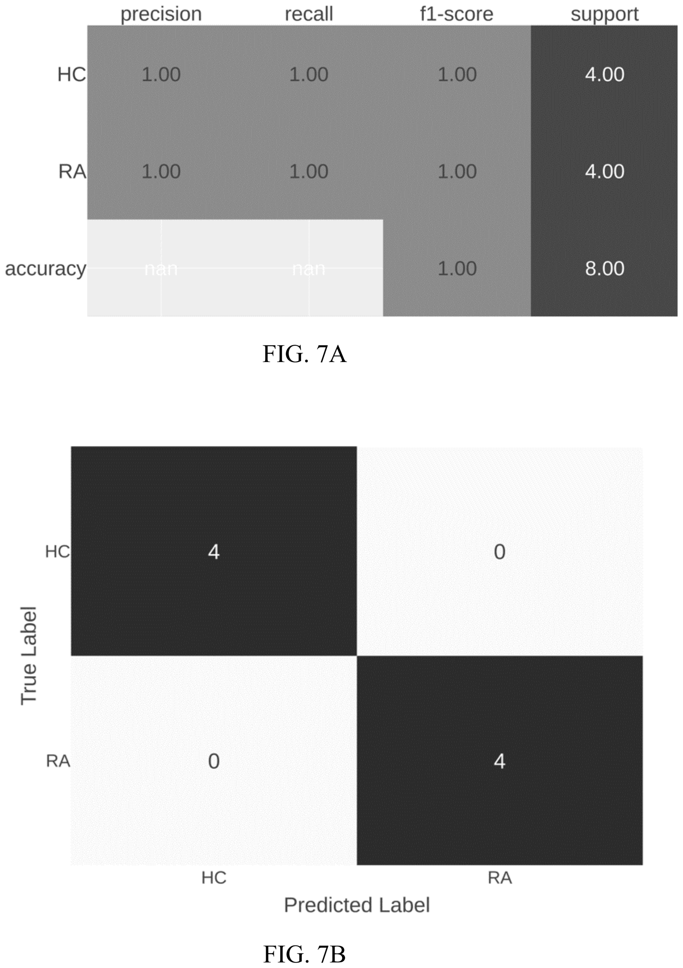

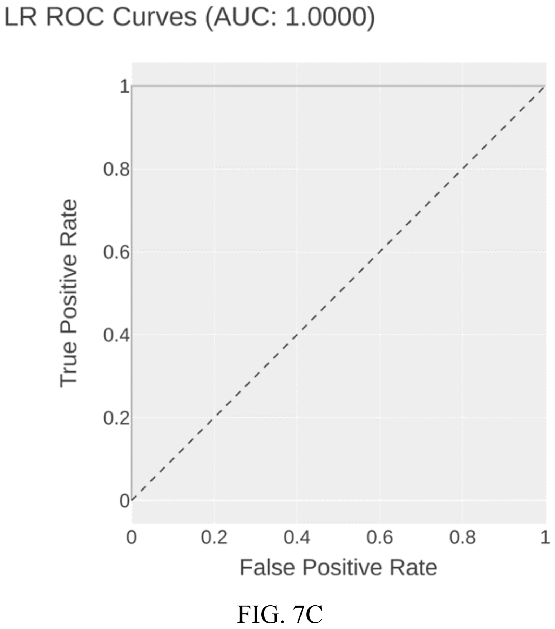

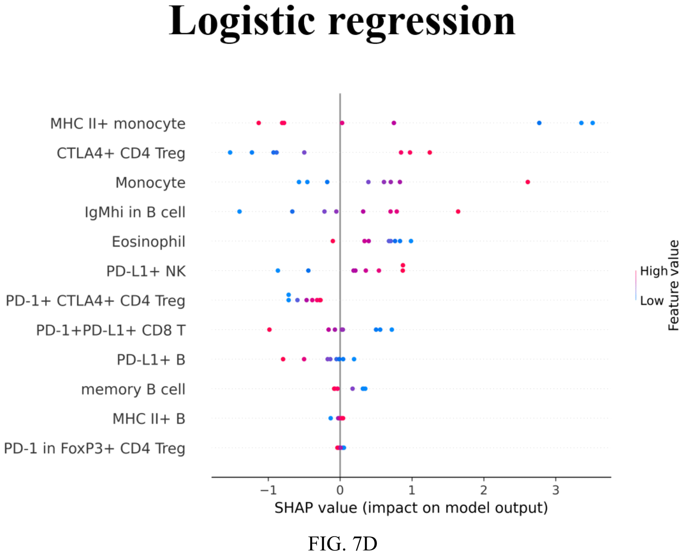

**FIGs. 7A-7D** illustrate an AI model can specifically discriminate immune cell profile between patients with RA and healthy controls, wherein after AI model training, we tested its performance in distinguishing ICPs between the RA and HC groups using **(****FIG. 7A****)** classification report, **(****FIG. 7B****)** confusion matrix, and **(****FIG. 7C****)** the receiver operating characteristic (ROC) analysis and interpreted the contribution of immune cell subsets in the discrimination by **(****FIG. 7D****)** a SHapley Additive exPlanations (SHAP) method.

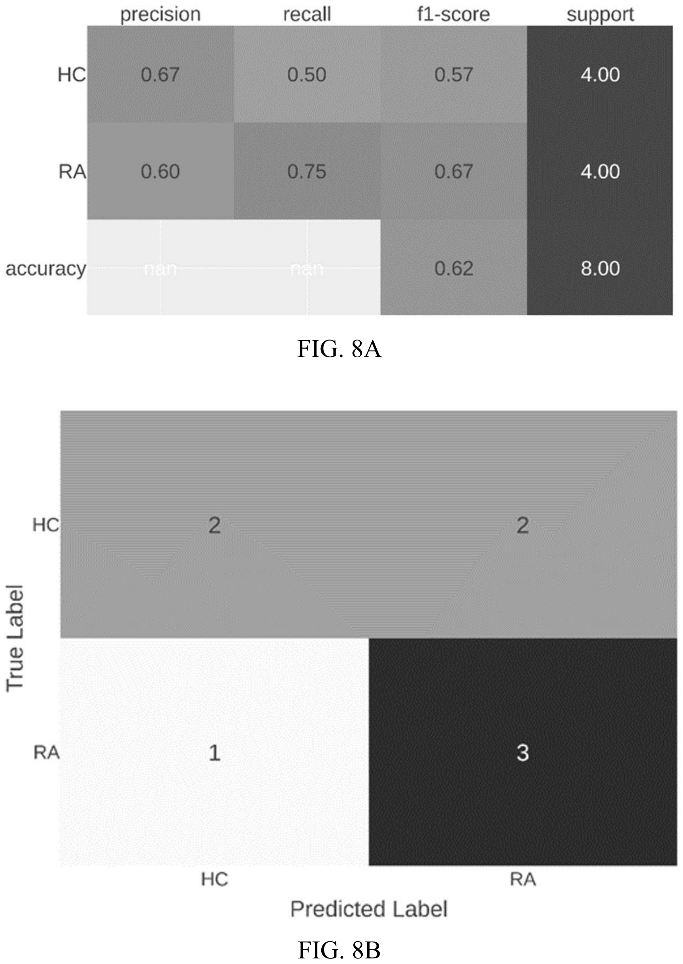

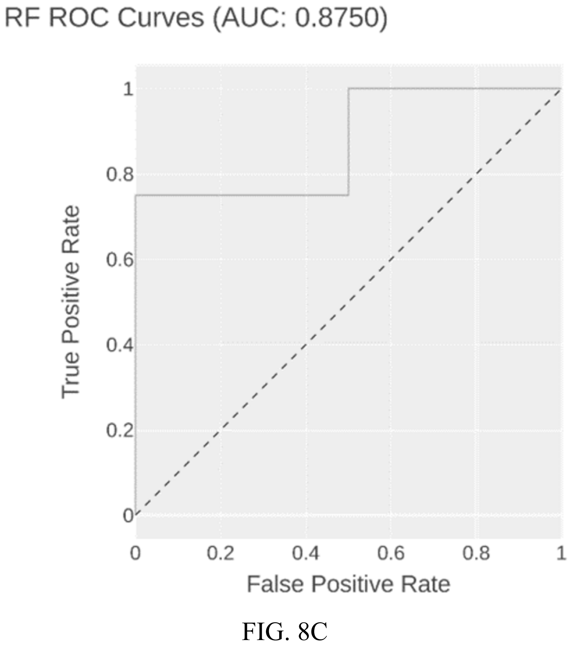

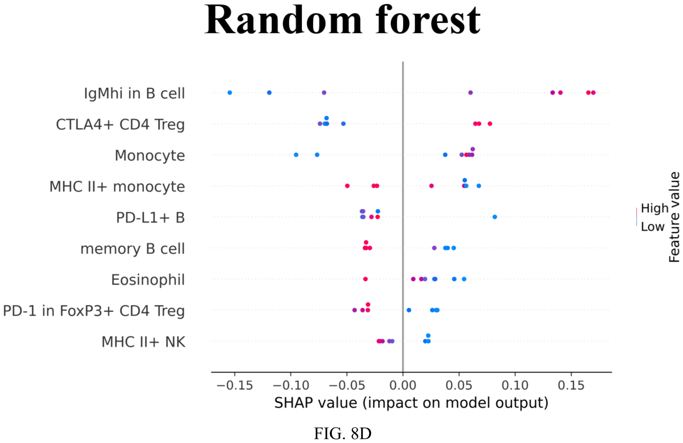

**FIGs. 8A-8D** illustrate a logistic regression (LR) algorithm showed superior performance in discrimination of immune cell profile than a random forest (RF) algorithm, wherein the machine learning algorithm was changed from the LR algorithm to the RF algorithm and conducted AI model training; after training the AI model, the discriminative performance of the AI model was tested using **(****FIG. 8A****)** a classification report and **(****FIG. 8B****)** a confusion matrix, and **(****FIG. 8C****)** the ROC analysis and interpreted the contribution of immune cell subsets in the discrimination by **(****FIG. 8D****)** a SHAP method.

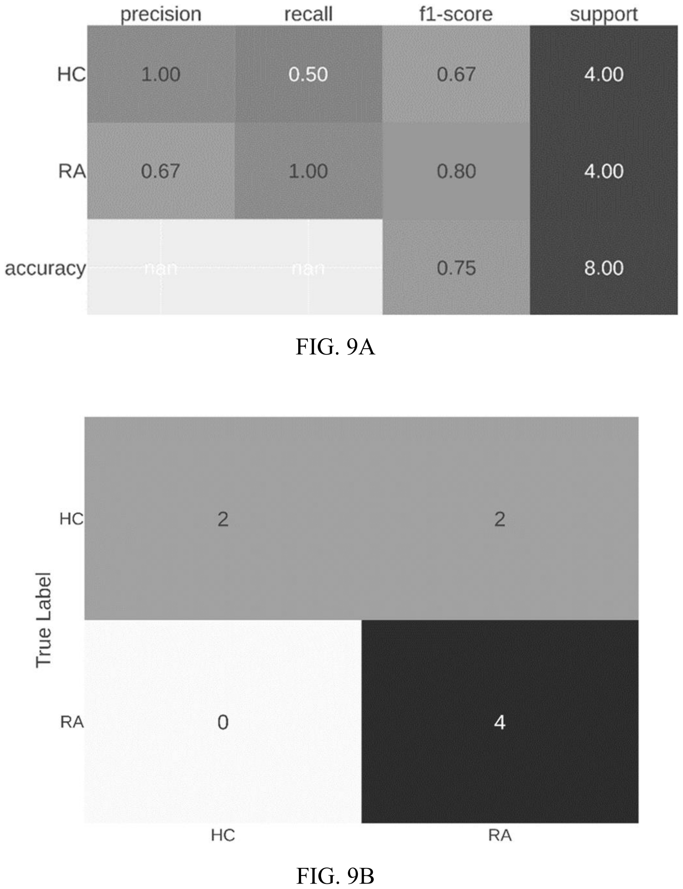

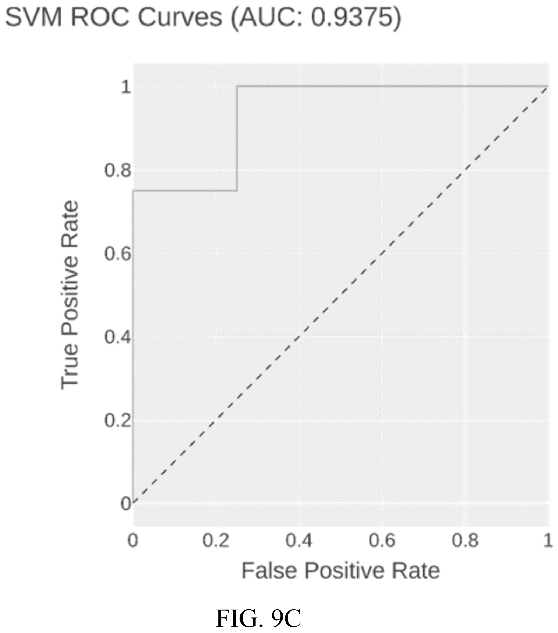

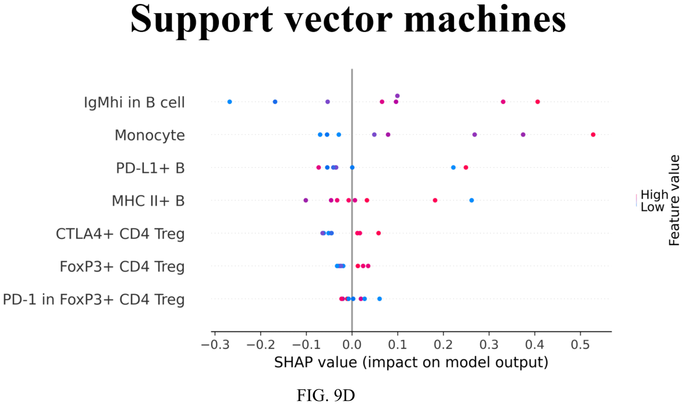

**FIGs. 9A-9D** illustrate the LR algorithm showed superior performance in discrimination of immune cell profile than a support vector machine (SVM) algorithm, wherein the machine learning algorithm was changed from the LR algorithm to the SVM algorithm and conducted AI model training; after training the AI model, the discriminative performance of the AI model was tested using **(****FIG. 9A****)** a classification report and **(****FIG. 9B****)** a confusion matrix, and **(****FIG. 9C****)** the ROC analysis and interpreted the contribution of immune cell subsets in the discrimination by **(****FIG. 9D****)** a SHAP method.

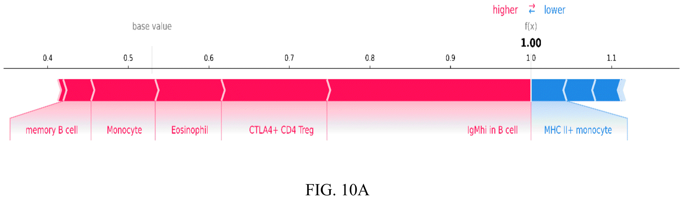

**FIG. 10A** illustrates a SHAP force plot that breaks down the evaluation into individual feature contributions, offering an intuitive visualization of how each feature influences a single evaluation.

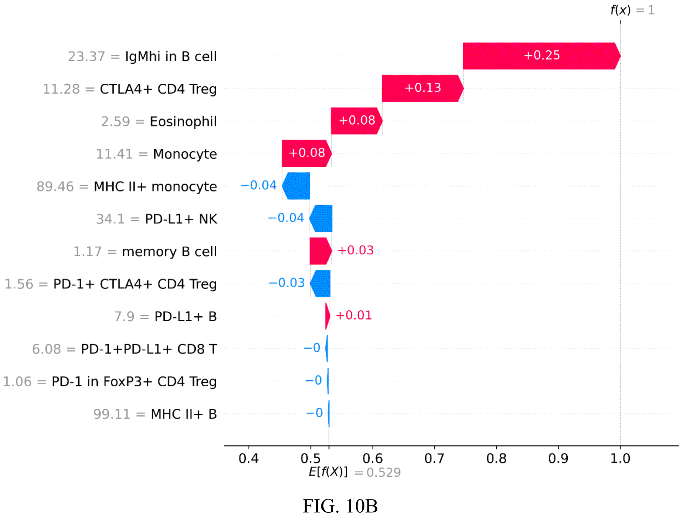

**FIG. 10B** illustrates a SHAP waterfall plot, showing how each feature contributes to the evaluation, highlighting their cumulative impact, where red represents a positive contribution to RA disease risk, while blue indicates a negative contribution; the final evaluation is the sum of all contributions.

### DETAILED DESCRIPTION

### Definitions

The term "healthy control" as used herein refers to a subject without a disease of rheumatoid arthritis (RA) being studied but may have other conditions indirectly affecting outcome.

The term "characterized immune cell subset" as used herein refers to that a group of immune cell subsets having a comparative immune profiling between RA patients and healthy controls (HCs), such as amounts of the immune cell subsets in the RA patients distinguishable from those in the HCs.

The term "predicted probability" as used as herein refers to the probability of a subject that is classified into a risk group for the RA disease.

The term "decision function" as used herein refers to a function that calculates the distance of the subject to the separating hyperplane of SVM classifier.

The term "hold-out set" as used herein is also called as "test set" and refers to the dataset not used in the training process of an AI model.

### Embodiments

In an embodiment, a method of processing biomedical data to generate a probability-based risk indicator for assessing the likelihood of a RA disease risk in a subject, includes steps of:
(a) staining peripheral blood mononuclear cells (PBMCs) and/or white blood cells (WBCs) from the subject by using a staining kit, wherein the staining kit includes:
   a first pattern, including antibodies against CD3, CD4, CD8, CD14, CD19, CD25, CD45, CD56, CTLA4, FoxP3 and PD-1;
   a second pattern, including antibodies against CD3, CD4, CD14, CD19, CD45, CD56, HLA-DR, PD-1 and PD-L1;
   a third pattern, including antibodies against CD3, CD11b, CD14, CD16, CD19, CD45, CD56, CD66b, CD123 and CD193; and
   a fourth pattern, including antibodies against CD10, CD19, CD21, CD23, CD38, CD45, CD127, IgG and IgM,
   wherein the antibodies of each pattern are labeled with fluorescent dyes;
(b) performing biomedical data acquisition of fluorescent intensity of each antibody bound to characterized immune cell subsets of an RA disease by using flow cytometry to obtain a dataset including data related to types of the characterized immune cell subsets and proportions thereof; and
(c) evaluating the dataset by using an artificial intelligent (AI) model to generate the probability-based risk indicator for the subject, thereby stratifying the subject into a risk category for the RA disease, wherein the generated probability-based risk indicator reflects the likelihood of the subject being at risk for the RA disease, providing a basis for further clinical evaluation or monitoring.

In an embodiment, the use of AI model comprises applying a machine learning method or algorithm based on a logistic regression (LR) algorithm.

In an embodiment, said step (c) of evaluating the dataset may include the following steps performed by the AI model:
generating the probability-based risk indicator based on a predicted probability derived from the machine learning algorithm utilizing the LR algorithm, and
outputting the probability-based risk indicator as an assessment result that classifies the subject into a risk group for the RA disease when the predicted probability meets or exceeds a predefined risk threshold value.

In an embodiment, the predefined risk threshold value of the predicted probability may be equal to 0.5-1.0. In an embodiment, the predefined risk threshold value may be 0.5, 0.55, 0.6, 0.65. 0.7, 0.75, 0.8, 0.85, 0.90, 0.95 or 1.0, preferably 0.5.

In an embodiment, the characterized immune cell subsets of the RA disease may include one, at least one, several or all, of eosinophil, memory B cell, IgM^{hi} in B cell, MHC II⁺ B cell, monocyte, MHC II⁺ monocyte, PD-L1⁺ NK cell, PD-L1⁺ B cell, PD-1⁺ PD-L1⁺ CD8 T cell, CTLA4⁺ CD4 T_{reg} cell, PD-1⁺ FoxP3⁺ CD4 T_{reg} cell, and PD-1⁺ CTLA4⁺ CD4 T_{reg} cell.

In an embodiment, the characterized immune cell subsets of the RA disease may be identified by steps of:
(i) staining the PBMCs and/or the WBCs of a plurality of healthy controls and a plurality of patients having the RA disease, respectively, by using a second staining kit (*e.g.,* as shown in **Table 3**);
(ii) performing data acquisition of fluorescent intensity of each antibody bound to the PBMCs and/or the WBCs of the healthy controls and the patients, respectively, by using flow cytometry;
(iii) identifying immune cell subsets in the PBMCs and/or the WBCs of the healthy controls and the patients, respectively, by using a pedigree method to obtain a dataset including data related to types of the immune cell subsets and proportions thereof;
(iv) performing data preprocessing of the dataset; and
(v) evaluating the preprocessed dataset by using the AI model to obtain immune cell subsets of the patients distinguishable from those of the healthy controls as the characterized immune cell subsets of the RA disease.

In an embodiment, the step (v) of evaluating the preprocessed dataset includes the following steps performed by the AI model:
performing a feature selection from the preprocessed data by using a Boruta algorithm to obtain predetermined immune cell subsets of the RA disease; and
applying data of the predetermined immune cell subsets to train the AI model with a machine learning algorithm selected from at least one of a random forest (RF) algorithm, a logistic regression (LR) algorithm and a support vector machines (SVM) algorithm so as to determine the characterized immune cell subsets.
Preferably, the method according to the present invention, insofar as it relates to a staining of cells from a patient and the acquisition of biomedical data, e.g. fluorescence data, is performed in-vitro.
In a further aspect, the present invention also relates to a staining kit, comprising a first pattern comprising antibodies against CD3, CD4, CD8, CD14, CD19, CD25, CD45, CD56, CTLA4, FoxP3 and PD-1; a second pattern comprising antibodies against CD3, CD4, CD14, CD19, CD45, CD56, HLA-DR, PD-1 and PD-L1; a third pattern comprising antibodies against CD3, CD11b, CD14, CD16, CD19, CD45, CD56, CD66b, CD123 and CD193; and a fourth pattern comprising antibodies against CD10, CD19, CD21, CD23, CD38, CD45, CD127, IgG and IgM, wherein the antibodies of each pattern are labeled with fluorescent dyes.

### Examples

### Example 1: Determining Characterized Immune Cell Subsets of RA Disease

Please see **FIG. 1** for a flow chart of determining the characterized immune cell subsets of RA disease. The related steps thereof are described in detail below.

### Materials and Methods

### Ethical Statements and Subject Enrollment

RA patients and HCs are enrolled at Taipei Veteran General Hospital (VGHTPE, Taipei City, Taiwan) from July 2021 to May 2024. Eligible RA patients were aged 18 to 75 years, diagnosed according to clinical practice (either newly diagnosed, in remission, or with progressive disease), and free of human immunodeficiency virus, hepatitis B virus, hepatitis C virus, or *Treponema pallidum* infection. Patients with history of malignancies, congenital or genetic disorders, or transplantations were excluded. HC had the same inclusive and exclusive criteria with RA patients except for the clinical confirmation of RA disease. Additionally, HCs who received routine medications within the past 2 months, those on medication due to acute illness within the past 2 weeks, or who were pregnant were excluded. After signing the informed consent form, 10 mL of peripheral blood was collected from the participants for immune cell profile (ICP) analysis. The design of this study complied with the Declaration of Helsinki. The Institute Review Board of VGHTPE reviewed and approved the protocol of this study (approval no. 2021-02-001, February 2021).

### Reagents and Antibodies

All reagents and antibodies used in this study were listed in **Tables 1 and 2.** Reagents were from Cytiva (Marlborough, MA, USA), Lonza (Basel, Switzerland), and Sigma-Aldrich (Merck KGaA, Darmstadt, Germany). Antibodies were all from Beckman-Coulter (Brea, CA, USA), Biolegend (San Diego, CA, USA), and Thermo-Fisher (Waltham, MA, USA). Reagents and antibodies were aliquoted and stored under conditions recommended by manufacturers.

**Table 1: Reagents**

| **Name** | **Manufacture** | **Catalogue number** |
|---|---|---|
| ***For PBMC and granulocyte isolation*** | | |
| Ficoll-Paque^{™} PREMIUM medium 1.077 g/mL | Cytiva | 17544202 |
| Phosphate buffer saline | Lonza | BE17-516F |
| ACK lysis buffer | Biolegend | 420301 |

| ***For immunostaining*** | | |
|---|---|---|
| Bovine serum albumin | Sigma-Aldrich | A7030 |
| Sodium azide | Sigma-Aldrich | S2002 |
| Foxp3/Transcription Factor Staining Buffer Set | Thermo-Fisher | 00-5523-00 |

**Table 2: Antibodies and fluorescent dyes conjugated therewith**

| **Target** | **Conjugation** | **Host** | **Manufacture** | **Catalogu e number** |
|---|---|---|---|---|
| CCR7 (also called "CD197") | PE/Cy7 | Mouse | Biolegend | 353226 |
| CD3 | APC-AF750 | Mouse | Beckman Coulter | A66329 |
| CD3 | KO | Mouse | Beckman Coulter | B00068 |
| CD4 | APC-AF700 | Mouse | Beckman Coulter | B10824 |
| CD4 | PE-Cy7 | Mouse | Beckman Coulter | 6607101 |
| CD8 | KO | Mouse | Beckman Coulter | B00067 |
| CD8 | PB | Mouse | Biolegend | 301023 |
| CD10 | PE/Cy7 | Mouse | Biolegend | 312214 |
| CD11b | PE/Cy7 | Mouse | Beckman Coulter | A54822 |
| CD11c | APC | Mouse | Biolegend | 301614 |
| CD13 | PerCP/Cy5.5 | Mouse | Biolegend | 301714 |
| CD14 | APC-AF750 | Mouse | Beckman Coulter | A86052 |
| CD14 | PB | Mouse | Beckman Coulter | B00846 |
| CD14 | PC5.5 | Mouse | Beckman Coulter | A70204 |
| CD16 | KO | Mouse | Beckman Coulter | B00069 |
| CD19 | APC-AF750 | Mouse | Beckman Coulter | A78838 |
| CD21 | APC | Mouse | Biolegend | 354906 |
| CD23 | PE | Mouse | Biolegend | 338508 |
| CD25 | PE | Mouse | Biolegend | 302606 |
| CD25 | BB515 | Mouse | BD | 564467 |
| CD27 | PB | Mouse | Biolegend | 356414 |
| CD28 | PE/Cy5 | Mouse | Biolegend | 302910 |
| CD33 | FITC | Mouse | Beckman Coulter | IM1135U |
| CD38 | PerCP/Cy5.5 | Mouse | Biolegend | 356614 |
| CD39 | PE | Mouse | Biolegend | 328208 |
| CD40 | FITC | Mouse | Biolegend | 334306 |
| CD45 | ECD | Mouse | Beckman Coulter | A07784 |
| CD45RA | PerCP/Cy5.5 | Mouse | Biolegend | 304121 |
| CD45RO | APC | Mouse | Biolegend | 304210 |
| CD56 | APC-AF700 | Mouse | Beckman Coulter | B10822 |
| CD56 | APC/Cy7 | Mouse | Biolegend | 318332 |
| CD62L | PB | Mouse | Biolegend | 304825 |
| CD64 | A700 | Mouse | Biolegend | 305040 |
| CD66b | PB | Mouse | Biolegend | 305112 |
| CD69 | PB | Mouse | Biolegend | 310919 |
| CD123 | PE | Mouse | Biolegend | 306006 |
| CD127 | BV421 | Mouse | Biolegend | 351310 |
| CD193 | FITC | Mouse | Biolegend | 310720 |
| CD203c | APC | Mouse | Biolegend | 354906 |
| CTLA4 | PE | Mouse | Biolegend | 349906 |
| CX3CR1 | PE | Mouse | Biolegend | 341604 |
| Foxp3 | APC | Mouse | Thermo-Fisher | 17-4777-42 |
| HLA-DR (Also called "MHC II") | PB | Mouse | Beckman Coulter | A74781 |
| HLA-DR (Also called "MHC II") | KO | Mouse | Beckman Coulter | B00070 |
| IgG | BV510 | Mouse | BD | 563247 |
| IgM | A700 | Mouse | Biolegend | 314538 |
| PD-1 | A488 | Mouse | Biolegend | 329936 |
| PD-1 | PE | Mouse | Biolegend | 329906 |
| PD-1 | PerCP/Cy5.5 | Mouse | Biolegend | 329914 |
| PD-L1 | PE | Mouse | Biolegend | 329706 |
| Siglec-8 | PerCP/Cy5.5 | Mouse | Biolegend | 347108 |
| TCR_{αβ} | FITC | Mouse | Biolegend | 306705 |
| TCR_{γδ} | APC | Mouse | Biolegend | 331212 |
| LAG-3 | PerCP/Cy5.5 | Mouse | Biolegend | 369312 |
| TIM-3 | PB | Mouse | Biolegend | 345042 |

### Software

Kaluza analysis software (V2.3; for data acquisition and plotting) and Prism (V9.5.1, for statistical analysis and data plotting) were obtained from Beckman Coulter (Kaluza; Brea, CA, USA) and GraphPad Software (Prism; La Jolla, CA, USA). The AI model was constructed by Scikit-learn under Python. All necessary tools for AI model building are available on GitHub.

### PBMC and WBC Isolation and Immunostaining

EDTA-anticoagulated peripheral blood (abbreviated as whole blood in the following) was aliquoted into two in which one was for PBMC isolation and the other was for granulocyte isolation (i.e. WBC isolation).

For PBMC isolation, one part of whole blood mixed with an aliquot of PBS was loaded into Ficoll-preloaded centrifuged tubes with Ficoll Paque medium (density 1.077 g/mL, Cytiva, Marlborough, MA, USA) and subjected to density centrifugation at 400 ×g (X-15R, Beckman-Coulter) for 30 minutes at room temperature. PBMCs were collected from the buffy coat and washed by PBS followed by being centrifuged with 750 ×g for 7 minutes, and then re-suspended for stained buffer (0.5% bovine serum albumin/PBS with 0.02% (w/v) sodium azide) and went through immunostaining.

For WBC isolation, the ammonium-chloride-potassium (ACK) lysis method was applied for the lysis of red blood cells (RBCs) based on the protocol in the manual of the lysis buffer. Briefly, the other part of whole blood was mixed with RBC lysis buffer (20X volume; Biolegend, San Diego, CA, USA) and incubated at room temperature for 10 minutes, then centrifuged at 400 ×g for 5 minutes to collect leukocytes (i.e. WBCs) from the pellet. The WBCs was washed by PBS and re-suspended in staining buffer (0.5% bovine serum albumin and 0.02% sodium azide in PBS) for immunostaining.

All PBMCs and WBCs were stained with cell surface markers and a part of them were further stained with intracellular cell markers. All staining procedures were kept in dark. For surface marker staining, PBMCs and WBCs were directly incubated with desired antibodies labeled with fluorescent dyes in the staining kit (see **Tables 2 and 3**) for 10 minutes at 4°C. For intracellular marker staining, a part of surface marker-labeled PBMCs and WBCs were fixed and permeabilized using Foxp3/Transcription Factor Staining Buffer Set (eBioscience^{™}) with recommended protocol from manual. Later, permeabilized PBMCs and WBCs were stained with CTLA4 or Foxp3 for 30 minutes at room temperature. Afterward, stained PBMCs and WBCs were washed with staining buffer once and followed by analysis by flow cytometer.

**Table 3: Staining kit**

| **Codes of Patterns / Dyes** | **PT-64** | **PT-65** | **PT-66** | **PT-67** | **PT-68** |
|---|---|---|---|---|---|
| **FL1** | FITC-TCR_{αβ} | BB515-CD25 | FITC-CD33 | FITC-PD-1 | FITC-TCR_{αβ} |
| **FL2** | PE-CD25 | PE-CTLA4 | PE-CD39 | PE-PD-L1 | PE-PD-1 |
| **FL3** | ECD-CD45 | ECD-CD45 | ECD-CD45 | ECD-CD45 | ECD-CD45 |
| **FL4** | PerCP/Cy5.5 -PD-1 | PerCP/Cy5.5 -PD-1 | PerCP/Cy5.5-CD13 | PerCP/Cy5.5-CD14 | PE/Cy5-CD28 |
| **FL5** | PE/Cy7-CD4 | PE/Cy7-CD4 | PE/Cy7-CD11b | PE/Cy7-CD4 | PE/Cy7-CD4 |
| **FL6** | APC-TCR_{γδ} | APC-Foxp3 | APC-CD11c | APC-CD 11 c | APC-TCR_{γδ} |
| **FL7** | APC-AF700-CD56 | APC-AF700-CD56 | | APC-AF700-CD56 | APC-AF700-CD56 |
| **FL8** | APC-AF750-CD3 | | APC-AF750-CD3 | | APC-AF750-CD3 |
| | | | APC/Cy7-CD56 | | |
| | APC-AF750-CD14 | APC-AF750-CD14 | | | APC-AF750-CD14 |
| | APC-AF750-CD19 | APC-AF750-CD19 | APC-AF750-CD19 | APC-AF750-CD19 | APC-AF750-CD19 |
| **FL9** | PB-CD69 | PB-CD8 | PB-CD14 | PB-HLA-DR | PB-CD27 |
| **FL10** | KO-CD8 | KO-CD3 | KO-HLA-DR | KO-CD3 | KO-CD8 |

| **Codes of Patterns / Dyes** | **PT-83** | **PT-85** | **PT-86** | **PT-87** |
|---|---|---|---|---|
| **FL1** | FITC-CD193 | FITC-TCR_{αβ} | FITC-TCR_{αβ} | FITC-CD40 |
| **FL2** | PE-CD123 | PE-CX3CR1 | PE-CX3CR1 | PE-CD23 |
| **FL3** | ECD-CD45 | ECD-CD45 | ECD-CD45 | ECD-CD45 |
| **FL4** | PerCP/Cy5.5-Siglec-8 | PerCP/Cy5.5-CD45RA | PerCP/Cy5.5-LAG-3 | PerCP/Cy5.5-CD38 |
| **FL5** | PE/Cy7-CD11b | PE/Cy7-CD197 | PE/Cy7-CD197 | PE/Cy7-CD10 |
| **FL6** | APC-CD203c | APC-CD45RO | APC-CD45RO | APC-CD21 |
| **FL7** | A700-CD64 | APC-AF700-CD4 | APC-AF700-CD4 | A700-IgM |
| **FL8** | APC-AF750-CD3 | | | |
| | APC/Cy7-CD56 | APC/Cy7-CD56 | APC/Cy7-CD56 | |
| | APC-AF750-CD14 | APC-AF750-CD14 | APC-AF750-CD14 | |
| | APC-AF750-CD19 | APC-AF750-CD19 | APC-AF750-CD19 | APC-AF750-CD19 |
| **FL9** | PB-CD66b | PB-CD62L | PB-TIM-3 | PB-CD127 |
| **FL10** | KO-CD16 | KO-CD8 | KO-CD8 | BV510-IgG |

### Data Acquisition, Cell Definition, Cell Population List and Dataset

For data acquisition, the fluorescent intensity of PBMCs and WBCs was measured using a flow cytometer (Navios, Beckman Coulter), and raw data were collected through Kaluza analysis software V2.3 (Beckman Coulter). Using a pedigree method, a filtering approach with two-marker sets (parameters on the X-axis and Y-axis) was applied to define immune cell subsets. The definition of these cell subsets was listed in **Table 4.** As shown in **FIGs. 2a-2i****,** the detailed filtrating processes were performed based on **Table 4** to obtain the raw dataset associated with at least 110 types of immune cell subsets and proportions thereof. For detailed process, using CD4 T_{reg} (CD3⁺CD14⁻CD19⁻CD56⁻CD4⁺CD8⁻FoxP3⁺CD25⁺) as an example PBMCs undergo six filtering steps (see **FIG. 2b****):** step 1. CD45⁺/SS; step 2. Regular FS/SS; step 3. CD14, CD19⁻/FS; step 4. CD3⁺/CD56⁻; step 5. CD4⁺/CD8⁻; step 6. FoxP3⁺/CD25⁺. Finally, the cell population proportions were obtained. The proportion of target cells was presented as the ratio between target cells and corresponding parent cells. The same process was applied to other cell populations.

**Table 4: Definition of immune cell subsets with markers**

| **Name of cell subsets** | **Markers** |
|---|---|
| ***Granulocytes*** | |
| CD11b⁺ Lineage⁻ cell | CD3⁻CD14⁻CD19⁻CD56⁻CD11b⁺ |
| Neutrophil | CD3⁻CD14⁻CD19⁻CD56⁻CD11b⁺CD16⁺CD64⁻CD66b⁺CD123⁻CD193⁻ |
| Eosinophil | CD3⁻CD14⁻CD19⁻CD56⁻CD11b⁺CD16⁻CD66b⁺CD123⁻CD193⁺ |
| Basophil | CD3⁻CD14⁻CD19⁻CD56⁻CD11b⁺CD16⁻CD66b⁻CD123⁺CD193⁺ |

| ***NK cell*** | |
|---|---|
| NK cell | CD3⁻CD14⁻CD19⁻CD56⁺ |
| CD8 NK cell | CD3⁻CD14⁻CD19⁻CD56⁺CD4⁻CD8⁺ |
| DN NK cell | CD3⁻CD14⁻CD19⁻CD56⁺CD4⁻CD8⁻ |

| ***NKT cell*** | |
|---|---|
| NKT cell | CD3⁺CD14⁻CD19⁻CD56⁺ |
| CD4 NKT cell | CD3⁺CD14⁻CD19⁻CD56⁺CD4⁺ |
| CD8 NKT cell | CD3⁺CD14⁻CD19⁻CD56⁺CD4⁻ |

| ***Dendritic cell*** | |
|---|---|
| DC | CD3⁻CD14⁻CD19⁻CD56⁻CD11c⁺ |
| MHC II⁺ DC | CD3⁻CD14⁻CD19⁻CD56⁻CD11c⁺MHC II⁺ |

| ***B cell*** | |
|---|---|
| B cell | CD14⁻CD19⁺ |
| IgG⁺ in B cell | CD19⁺CD127⁻IgG⁺IgM⁻ |
| Long lived plasma cell | CD19⁺CD127⁻IgG⁺IgM⁻CD10⁺CD21⁺ |
| Germinal center B cell | CD19⁺CD127⁻IgG⁺IgM⁻CD10⁺CD21⁻ |
| Memory B cell | CD19⁺CD127⁻IgG⁺IgM⁻CD10⁻CD21⁺CD23⁻CD38⁺ |
| IgM^{dim} in B cell | CD19⁺CD127⁻IgG⁻IgM^{dim} |
| Follicular B cell | CD19⁺CD127⁻IgG⁻IgM^{dim}CD21⁺CD23⁺CD38⁺CD10⁻ |
| Short lived plasma cell | CD19⁺CD127⁻IgG⁻IgM^{dim}CD21⁺CD23⁻CD38⁺CD10⁻ |
| IgM^{hi} in B cell | CD19⁺CD127⁻IgG⁻IgM^{hi} |
| Marginal Zone B cell | CD19⁺CD127⁻IgG⁻IgM^{hi}CD21⁺CD10⁻CD38⁺CD23⁻ |
| Transitional B cell | CD19⁺CD127⁻IgG⁻IgM^{hi}CD21⁻CD10⁺CD38⁺CD23⁻ |
| MHC II⁺ B cell | CD14⁻CD19⁺MHC II⁺ |

| ***Monocyte*** | |
|---|---|
| Monocyte | CD14⁺CD19⁻ |
| MHC II⁺ monocyte | CD14⁺CD19⁻MHC II⁺ |

| ***T lymphocyte*** | |
|---|---|
| T cell | CD3⁺CD14⁻CD19⁻CD56⁻ |
| CD4 αβ T | CD3⁺CD14⁻CD19⁻CD56⁻TCR_{αβ}⁺TCR_{γδ}⁻CD4⁺CD8⁻ |
| Terminal CD4 αβ T | CD3⁺CD14⁻CD19⁻CD56⁻TCR_{αβ}⁺TCR_{γδ}⁻CD4⁺CD8⁻CD25⁻CD69⁺ |
| Immediately activated CD4 αβ T | CD3⁺CD14⁻CD19⁻CD56⁻TCR_{αβ}⁺TCR_{γδ}⁻CD4⁺CD8⁻CD27⁻CD28⁻ |
| Naïve CD4 αβ T | CD14⁻CD19⁻CD56⁻TCR_{αβ}⁺CD4⁺CD8⁻CD45RO⁻CCR7⁺ |
| Effector CD4 αβ T | CD14⁻CD19⁻CD56⁻TCR_{αβ}⁺CD4⁺CD8⁻CD45RO⁻CCR7⁻ |
| Exhausted effector CD4 αβ T | CD14⁻CD19⁻CD56⁻TCR_{αβ}⁺CD4⁺CD8⁻CD45RO⁻CCR7⁻TIM-3⁺LAG-3⁺ |
| Central memory CD4 αβ T | CD14⁻CD19⁻CD56⁻TCR_{αβ}⁺CD4⁺CD8⁻CD45RO⁺CCR7⁺ |
| Exhausted central memory CD4 αβ T | CD14⁻CD19⁻CD56⁻TCR_{αβ}⁺CD4⁺CD8⁻CD45RO⁺CCR7⁺TIM-3⁺LAG-3⁺ |
| Effector memory CD4 αβ T | CD14⁻CD19⁻CD56⁻TCR_{αβ}⁺CD4⁺CD8⁻CD45RO⁺CCR7⁻ |
| Exhausted effector memory CD4 αβ T | CD14⁻CD19⁻CD56⁻TCR_{αβ}⁺CD4⁺CD8⁻CD45RO⁺CCR7⁻TIM-3⁺LAG-3⁺ |
| CD8 αβ T | CD3⁺CD14⁻CD19⁻CD56⁻TCR_{αβ}⁺TCR_{γδ}⁻CD4⁻CD8⁺ |
| Terminal effector CD8 αβ T | CD3⁺CD14⁻CD19⁻CD56⁻TCR_{αβ}⁺TCR_{γδ}⁻CD4⁻CD8⁺CD25⁻CD69⁺ |
| Immediately activated CD8 αβ T | CD3⁺CD14⁻CD19⁻CD56⁻TCR_{αβ}⁺TCR_{γδ}⁻CD4⁻CD8⁺CD27⁻CD28⁻ |
| Naive CD8 αβ T | CD14⁻CD19⁻CD56⁻TCR_{αβ}⁺CD4⁻CD8⁺CD45RO⁻CCR7⁺ |
| Effector CD8 αβ T | CD14⁻CD19⁻CD56⁻TCR_{αβ}⁺CD4⁻CD8⁺CD45RO⁻CCR7⁻ |
| Exhausted effector CD8 αβ T | CD14⁻CD19⁻CD56⁻TCR_{αβ}⁺CD4⁻CD8⁺CD45RO⁻CCR7⁻TIM-3⁺LAG-3⁺ |
| Central memory CD8 αβ T | CD14⁻CD19⁻CD56⁻TCR_{αβ}⁺CD4⁻CD8⁺CD45RO⁺CCR7⁺ |
| Exhausted central memory CD8 αβ T | CD14⁻CD19⁻CD56⁻TCR_{αβ}⁺CD4⁻CD8⁺CD45RO⁺CCR7⁺TIM-3⁺LAG-3⁺ |
| Effector memory CD8 αβ T | CD14⁻CD19⁻CD56⁻TCR_{aβ}⁺CD4⁻CD8⁺CD45RO⁺CCR7⁻ |
| Exhausted effector memory CD8 αβ T | CD14⁻CD19⁻CD56⁻TCR_{αβ}⁺CD4⁻CD8⁺CD45RO⁺CCR7⁻TIM-3⁺LAG-3⁺ |
| CD8 γδ T | TCR_{αβ}⁻TCR_{γδ}⁺CD4⁻CD8⁺ |
| DN γδ T | TCR_{αβ}⁻TCR_{γδ}⁺CD4⁻CD8⁻ |

| ***PD-1⁺ cells*** | |
|---|---|
| PD-1⁺ NK | CD3⁻CD14⁻CD19⁻CD56⁺PD-1⁺PD-L1⁻ |
| PD-1⁺ CD4 NKT | CD3⁺CD14⁻CD19⁻CD56⁺CD4⁺PD-1⁺PD-L1⁻ |
| PD-1⁺ CD8 NKT | CD3⁺CD14⁻CD19⁻CD56⁺CD4⁻PD-1⁺PD-L1⁻ |
| PD-1⁺ DC | CD3⁻CD14⁻CD19⁻CD56⁻CD11c⁺PD-1⁺PD-L1⁻ |
| PD-1⁺ monocyte | CD14⁺CD19⁻PD-1⁺PD-L1⁻ |
| PD-1⁺ B | CD14⁻CD19⁺ PD-1⁺PD-L1⁻ |
| PD-1⁺ CD4 T | CD3⁺CD14⁻CD19⁻CD56⁻CD4⁺PD-1⁺PD-L1⁻ |
| PD-1⁺ CD8 T | CD3⁺CD14⁻CD19⁻CD56⁻CD4⁻PD-1⁺PD-L1⁻ |

| ***PD-L1***⁺ ***cells*** | |
|---|---|
| PD-L1⁺ NK | CD3⁻CD14⁻CD19⁻CD56⁺PD-1⁻PD-L1⁺ |
| PD-L1⁺ CD4 NKT | CD3⁺CD14⁻CD19⁻CD56⁺CD4⁺PD-1⁻PD-L1⁺ |
| PD-L1⁺ CD8 NKT | CD3⁺CD14⁻CD19⁻CD56⁺ CD4⁻PD-1⁻PD-L1⁺ |
| PD-L1⁺ DC | CD3⁻CD14⁻CD19⁻CD56⁻CD11c⁺PD-1⁻PD-L1⁺ |
| PD-L1⁺ monocyte | CD14⁺CD19⁻PD-1⁻PD-L1⁺ |
| PD-L1⁺ B | CD14⁻CD19⁺ PD-1⁻PD-L1⁺ |
| PD-L1⁺ CD4 T | CD3⁺CD14⁻CD19⁻CD56⁻CD4⁺PD-1⁻PD-L1⁺ |
| PD-L1⁺ CD8 T | CD3⁺CD14⁻CD19⁻CD56⁻CD4⁻PD-1⁻PD-L1⁺ |

| ***PD-1⁺ PD-L1⁺ cells*** | |
|---|---|
| PD-1⁺ PD-L1⁺ NK | CD3⁻CD14⁻CD19⁻CD56⁺PD-1⁺PD-L1⁺ |
| PD-1⁺ PD-L1⁺ CD4 NKT | CD3⁺CD14⁻CD19⁻CD56⁺CD4⁺PD-l⁺PD-L1⁺ |
| PD-1⁺ PD-L1⁺ CD8 NKT | CD3⁺CD14⁻CD19⁻CD56⁺CD4⁻PD-1⁺PD-L1⁺ |
| PD-1⁺ PD-L1⁺ DC | CD3⁻CD14⁻CD19⁻CD56⁻CD11c⁺PD-1⁺PD-L1⁺ |
| PD-1⁺ PD-L1⁺ monocyte | CD14⁺CD19⁻PD-1⁺PD-L1⁺ |
| PD-1⁺ PD-L1⁺ B | CD14⁻CD19⁺ PD-1⁺PD-L1⁺ |
| PD-1⁺ PD-L1⁺ CD4 T | CD3⁺CD14⁻CD19⁻CD56⁻CD4⁺PD-1⁺PD-L1⁺ |
| PD-1⁺ PD-L1⁺ CD8 T | CD3⁺CD14⁻CD19⁻CD56⁻CD4⁻PD-1⁺PD-L1⁺ |

| ***Regulatory cells*** | |
|---|---|
| Foxp3⁺ CD4 T_{reg} | CD3⁺CD14⁻CD19⁻CD56⁻CD4⁺CD8⁻FoxP3⁺CD25⁺ |
| Foxp3⁺ CD8 T_{reg} | CD3⁺CD14⁻CD19⁻CD56⁻CD4⁻CD8⁺FoxP3⁺CD25⁺ |
| Foxp3⁺ CD8 NK_{reg} | CD3⁻CD14⁻CD19⁻CD56⁺CD4⁻CD8⁺FoxP3⁺CD25⁺ |

| ***PD-1*⁺ *Regulatory cells*** | |
|---|---|
| Foxp3⁺ DN NK_{reg} | CD3⁻CD14⁻CD19⁻CD56⁺CD4⁻CD8⁻FoxP3⁺CD25⁺ |
| Foxp3⁺ CD4 NKT_{reg} | CD3⁺CD14⁻CD19⁻CD56⁺CD4⁺CD8⁻FoxP3⁺CD25⁺ |
| Foxp3⁺ CD8 NKT_{reg} | CD3⁺CD14⁻CD19⁻CD56⁺CD4⁻CD8⁺FoxP3⁺CD25⁺ |
| CTLA4⁺ CD4 T_{reg} | CD3⁺CD14⁻CD19⁻CD56⁻CD4⁺CD8⁻CTLA4⁺CD25⁺ |
| CTLA4⁺ CD8 T_{reg} | CD3⁺CD14⁻CD19⁻CD56⁻CD4⁻CD8⁺CTLA4⁺CD25⁺ |
| CTLA4⁺ CD8 NK_{reg} | CD3⁻CD 14⁻CD 19⁻CD56⁺CD4⁻CD8⁺CTLA4CD25⁺ |
| CTLA4⁺ DN NK_{reg} | CD3⁻CD14⁻CD19⁻CD56⁺CD4⁻CD8⁻CTLA4⁺CD25⁺ |
| CTLA4⁺ CD4 NKT_{reg} | CD3⁺CD14⁻CD19⁻CD56⁺CD4⁺CD8⁻CTLA4⁺CD25⁺ |
| CTLA4⁺ CD8 NKT_{reg} | CD3⁺CD14⁻CD19⁻CD56⁺CD4⁻CD8⁺CTLA4⁺CD25⁺ |
| HLA-DR⁻ MDSC | CD3⁻CD14⁺CD19⁻CD56⁻CD11b⁺HLA-DR⁻ |
| HLA-DR^{dim} MDSC | CD3⁻CD14⁺CD19⁻CD56⁻CD11b⁺HLA-DR^{dim} |
| MHC II⁺ NK | CD3⁻CD14⁻CD19⁻CD56⁺MHC II⁺ |
| MHC II⁺ CD4 NKT | CD3⁺CD14⁻CD19⁻CD56⁺CD4⁺MHC II⁺ |
| MHC II⁺ CD8 NKT | CD3⁺CD14⁻CD19⁻CD56⁺CD4⁻MHC II⁺ |
| MHC II⁺ CD4 T | CD3⁺CD14⁻CD19⁻CD56⁻CD4⁺MHC II⁺ |
| MHC II⁺ CD8 T | CD3⁺CD14⁻CD19⁻CD56⁻CD4⁻MHC II⁺ |
| CTLA4 in FoxP3⁺ CD4 T_{reg} | CD3⁺CD14⁻CD19⁻CD56⁻CD4⁺CD8⁻FoxP3⁺CD25⁺ CTLA4⁺ |
| PD-1 in FoxP3⁺ CD4 T_{reg} | CD3⁺CD14⁻CD19⁻CD56⁻CD4⁺CD8⁻FoxP3⁺CD25⁺ PD-1⁺ |
| CTLA4⁺ PD-1⁺ in FoxP3⁺ CD4 T_{reg} | CD3⁺CD14⁻CD19⁻CD56⁻CD4⁺CD8⁻FoxP3⁺CD25⁺ CTLA4⁺PD-1⁺ |
| CTLA4 in FoxP3⁺ CD8 T_{reg} | CD3⁺CD14⁻CD19⁻CD56⁻CD4⁻CD8⁺FoxP3⁺CD25⁺ CTLA4⁺ |
| PD-1 in FoxP3⁺ CD8 T_{reg} | CD3⁺CD14⁻CD19⁻CD56⁻CD4⁻CD8⁺FoxP3⁺CD25⁺ PD-1⁺ |
| CTLA4⁺ PD-1⁺ in FoxP3⁺ CD8 T_{reg} | CD3⁺CD14⁻CD19⁻CD56⁻CD4⁻CD8⁺FoxP3⁺CD25⁺ CTLA4⁺PD-1⁺ |
| PD-1⁺ Foxp3⁺ CD4 T_{reg} | CD3⁺CD14⁻CD19⁻CD56⁻CD4⁺CD8⁻FoxP3⁺CD25⁺PD-1⁺ |
| PD-1⁺ Foxp3⁺ CD8 T_{reg} | CD3⁺CD14⁻CD19⁻CD56⁻CD4⁻CD8⁺FoxP3⁺CD25⁺PD-1⁺ |
| PD-1⁺ Foxp3⁺ CD8 NK_{reg} | CD3⁻CD14⁻CD19⁻CD56⁺CD4⁻CD8⁺FoxP3⁺CD25⁺PD-1⁺ |
| PD-1⁺Foxp3⁺ DN NK_{reg} | CD3⁻CD14⁻CD19⁻CD56⁺CD4⁻CD8⁻FoxP3⁺CD25⁺PD-1⁺ |
| PD-1⁺ Foxp3⁺ CD4 NKT_{reg} | CD3⁺CD14⁻CD19⁻CD56⁺CD4⁺CD8⁻FoxP3⁺CD25⁺PD-1⁺ |
| PD-1⁺ Foxp3⁺ CD8 NKT_{reg} | CD3⁺CD14⁻CD19⁻CD56⁺CD4⁻CD8⁺FoxP3⁺CD25⁺PD-1⁺ |
| PD-1⁺ CTLA4⁺ CD4 T_{reg} | CD3⁺CD14⁻CD19⁻CD56⁻CD4⁺CD8⁻CTLA4⁺CD25⁺PD-1⁺ |
| PD-1⁺ CTLA4⁺ CD8 T_{reg} | CD3⁺CD14⁻CD19⁻CD56⁻CD4⁻CD8⁺CTLA4⁺CD25⁺PD-1⁺ |
| PD-1⁺ CTLA4⁺ CD8 NK_{reg} | CD3⁻CD14⁻CD19⁻CD56⁺CD4⁻CD8⁺CTLA4CD25⁺PD-1⁺ |
| PD-1⁺ CTLA4⁺ DN NK_{reg} | CD3⁻CD14⁻CD19⁻CD56⁺CD4⁻CD8⁻CTLA4⁺CD25⁺PD-1⁺ |
| PD-1⁺ CTLA4⁺ CD4 NKT_{reg} | CD3⁺CD14⁻CD19⁻CD56⁺CD4⁺CD8⁻CTLA4⁺CD25⁺PD-1⁺ |
| PD-1⁺ CTLA4⁺ CD8 NKT_{reg} | CD3⁺CD14⁻CD19⁻CD56⁺CD4⁻CD8⁺CTLA4⁺CD25⁺PD-1⁺ |

| | |
|---|---|
| *Abbreviation: DC, dendritic cell; DN, double negative; MHC II, major histocompatibility complex class II; NK, natural killer cell; NKT, natural killer T cell; PD-1, programmed cell death 1; PD-L1, programmed cell death ligand 1; TCR, T cell receptor. | |

### Classical Statistical Analysis for Determining Characterized Immune Cell Subsets

Classical statistics employs the Mann-Whitney U test, which is used to determine whether there is a difference in the medians between two independent samples. Through this method, it can be observed that immune system changes vary uniquely across different diseases, with statistically significant cell populations differing accordingly.

The chi-square test or Fisher exact test was used to compare categorical variables, and the Mann-Whitney U-test was used to compare numerical data. Statistical significance was set at p < 0.05. All analyses were performed using SPSS software (version 26.0, IBM SPSS Statistics for Windows; IBM, Armonk, New York, USA). The proportion of each immune cell is shown in a bar chart that was plotted using Prism version 9.5.1 (GraphPad Software Inc., La Jolla, CA, USA). The columns with statistical differences between the RA and HC groups (p-values of the Mann-Whiteney-U test lower than 0.05, 0.01, 0.001, or 0.0001) are labeled with *, **, ***, or ****.

### AI Model Developed Using Python for Determining Characterized Immune Cell Subsets

Please see **FIG. 3****,** which illustrates a flow chart of an AI model training developed using Python and trained with a machine learning algorithm using scikit-learn. In detail, the raw dataset described above consisted of HC group (n = 21) and RA group (n = 21). Data preprocessing was performed by the following steps. First, the target labels were converted to the numerical value 0 for HC and the numerical value 1 for RA. 64 types of immune cell subsets were reserved for each subject after data preprocessing. The hold-out set (i.e. test set) consisted of some HC subjects (n=4), which were split from the 21 HC subjects, and some RA subjects (n=4), which were split from the 21 RA subjects. The remaining HC subjects (n=17) and the remaining RA subjects (n=17) were used as the training set. Feature selection in the training set was performed using the Boruta algorithm, which is a wrapper-based technique based on the random forest algorithm. Boruta compared the Z-scores of the shuffled shadow features and the original features to determine feature importance in every iteration. After the predefined iterations, predetermined immune cell subsets of the disease were obtained, which are more significantly relevant to classification than randomly permuted features.

The training set with the predetermined immune cell subsets was applied to train the AI model using three different types of machine learning algorithms - Random forest (RF), logistic regression (LR), and support vector machines (SVM) algorithms, which were used to classify flow cytometry data from 2 classes of HC and RA subjects. For SVM, the Min-Max scaling range obtained from the training process was applied to the hold-out set to ensure that there is no data leakage. To minimize the impact of the immune cell subset with a relatively higher magnitude on the distance calculation, the Min-Max scaling was applied for SVM to ensure that every immune cell subset had a similar effect when the classifier constructed the hyperplane. The Min-Max scaling is a normalization technique that transforms the minimal immune cell subset value to 0 and the maximal immune cell subset value to 1.

The sensitivity and specificity of the trained AI model were then tested using the hold-out set. If the predicted probability of the trained AI model using RF or LR algorithm was greater than 0.5, the AI model predicted the subject as RA disease. If the decision function of the trained AI model using SVM algorithm was greater than 0, the AI model predicted the subject as RA disease. The discriminative ability of the models was then evaluated by the area under curve (AUC) of the receiver operating characteristic (ROC) curve. The ROC curve is often used to compare the model performance in a clinical classification problem. It shows the relation of the true positive rate (sensitivity) against the false positive rate (1-specificity) for each possibility threshold. The better the discriminative ability of the AI model, the closer the ROC curve is to the upper left corner of the plot. Finally, the discriminative ability of the AI model was quantified by computing the area under the ROC curve using the trapezoidal rule to obtain the AUC results. The ROC curves and the AUC results of the training set were visualized to compare the AI model performance. The Shapley Additive exPlanations (SHAP) was applied to explain the AI model by computing the contribution of each predetermined immune cell subset to prediction. The SHAP summary plot was depicted to visualize the ranking of immune cell subset importance and the value of the feature per subject with the SHAP values. The color of the data point in each immune cell subset represents a high or a low feature value, in which each data point represents one subject. Red indicates the high value of immune cell subset, and blue indicates the low value of immune cell subset. The y-axis of the plot is the immune cell subset importance ranking of the predetermined immune cell subsets, and the x-axis is the SHAP value range. According to the trained AI model, the higher the SHAP value, the higher the risk of RA disease. Finally, the characterized immune cell subsets can be obtained via said AI model training.

### Results

### Baseline information on the participated subjects

The demographic information of the 21 newly diagnosed RA patients and HCs was shown in **Table 5,** with a male-to-female ratio of 1:3.2 in the RA group and 1:2 in the HC group. The median age at enrolment in RA and HC groups was 53 years and 58 years, respectively (*p* = 0.59). In the RA group, 14 (67%) were seropositive for ACPA, 16 (76%) were seropositive for RF. Mean ESR was 26.5 ± 26.3 mm/hour, while Median serum CRP protein level was 0.28 mg/mL.

**Table 5:**

| | **RA (N = 21)** | **HC (N = 21)** | ***p*** |
|---|---|---|---|
| Female, N (%) | 16 (76%) | 14 (67%) | 0.1392 |
| Age at diagnosis of RA, years (median, range) | 53 (26 - 75) | 58 (31 - 74) | 0.5879 |
| Factors associated with RA | | | |
| ACPA positive, N (%) | 14 (67%) | | |
| RF positive, N (%) | 16 (76%) | | |
| Baseline ESR, mm/hour (mean, SD) | 26.5 (26.3) | | |
| Baseline CRP, mg/dL (median, range) | 0.28 (0 - 4.62) | | |

| | | | |
|---|---|---|---|
| *Abbreviation: ACPA, anti-citrullinated peptide antibody; CRP, C-reactive protein; ESR, erythrocyte sedimentation rate; HC, healthy control; SD, standard deviation; RA, rheumatoid arthritis; RF, rheumatoid factor. | | | |

### Characterization of the ICP

ICP with cellular immunity (monocytes, dendritic cells, natural killer cells, and CD8 T cells), humoral immunity (major histocompatibility complex class II-positive [MHC II⁺] cells, CD4 T cells and B cells), innate immunity (granulocytes), and immune tolerance (regulatory cells, PD-1/PD-L1⁺ cells, and MDSCs) was analyzed.

16 immune cell subsets (see **Table 6)** were identified using classical statistics with their proportions in RA patients significantly differing than those in the HCs, which was described in detail in the following paragraphs.

**Table 6: Identified immune cell subsets by classical statistics**

| **Mann-Whitney U test** | **Immune cell subsets** |
|---|---|
| **** (p < 0.0001) | IgM^{hi} in B cell, Monocyte |
| *** (p < 0.001) | - |
| ** (p < 0.01) | Memory B cell, CD11b⁺lineage⁻ cell, Eosinophil, MHC II⁺ Monocyte, CTLA4⁺ CD4 T_{reg}, CTLA4 in FoxP3⁺ CD4 T_{reg} |
| * (p < 0.05 | Marginal Zone B cell, FoxP3⁺ CD4 T_{reg} cell, MHC II⁺ CD4 T cell, MHC II⁺ CD8 T cell, MHC II⁺ NK cell, PD-L1⁺ NK cell, PD-L1⁺ CD8 NKT cell, PD-1⁺ PD-L1⁺ CD4 T cell |

### RA patients exhibited activated humoral and innate immunity (for classical statistics Mann-Whitney U test)

In the investigation of effector cells in RA patients, the proportions of CD4 and CD8 T-cell lineages-particularly effector, central memory, and effector memory T cells-showed an increasing trend in the RA group compared to the HC group, suggesting an activated trend presented in cellular immunity **(****FIG. 4**). For humoral immunity, proportions of marginal zone B cells (*p* = 0.01) and IgM^{hi} subpopulation in B cells (*p* < 0.0001) were significantly higher in the RA group compared to the HC group **(****FIG. 5A****).** In contrast, the proportion of memory B cells in the RA group showed a significant decrease compared to the HC group (*p* = 0.001; **FIG. 5A****).** A total of 67% and 76% of the subjects in the RA group had respectively positive results in the ACPA and RF assay **(Table 5).** Given that ACPA and RF are autoantibodies against citrullinated fibrinogen and the Fc domain of endogenous antibodies, the activation of humoral immunity observed in RA patients is therefore foreseeable.

In addition to cellular and humoral immunity, significantly increased proportions of CD11b⁺lineage⁻ cells (*p =* 0.007), monocytes (*p* = 0.0001), and its MHC⁺ subpopulation (*p* = 0.002) were observed in the RA group compared to the HC group, whereas the proportion of eosinophils in the RA group was significantly lower than that in the HC group (*p* = 0.001; **FIG. 5B****)**. Since CD11b⁺lineage⁻ cells include granulocytes, monocytes, and macrophages, and neutrophils and basophils proportions in the RA group were comparable to those in the HC group (data not shown), the increase of CD11b⁺lineage⁻ cells may be attributed to monocyte and macrophage increase. Summarizing the above results, the RA patients had activated humoral and innate immunity.

### RA patients showed compensatively increased immune tolerance (for classical statistics_Mann-Whitney U test)

The changes in immune tolerance between RA patients and HCs were evaluated subsequently. The proportions of the CTLA4⁺ regulatory CD4 T cells (CTLA4⁺CD25⁺; *p =* 0.003), FoxP3⁺ regulatory CD4 T cells (FoxP3⁺CD25⁺; *p* = 0.02), and CTLA4⁺ subpopulation of FoxP3⁺ regulatory CD4 T cells (FoxP3⁺CD25⁺ CTLA4⁺; *p* = 0.009) were significantly higher in the RA group than in the HC group **(****FIG. 6A****).** For PD-1⁺/PD-L1⁺ cells, the proportions of PD-L1⁺ subpopulations of natural killer (NK) cells *(p* = 0.03), CD8 NKT cells *(p* = 0.02), and PD-1⁺ CD4 T cells (*p* = 0.04) were significantly higher in the RA group compared to those in the HC group **(****FIG. 6B****).** For MHC II⁺ cells, the proportions of CD4 T, (*p =* 0.01) and CD8 T (*p* = 0.03) subpopulations (*p* = 0.002) were significantly higher in the RA group than the HC group **(****FIG. 6C****).** Of note, the proportion of MHC II⁺ NK cells was significantly lower in the RA group than in the HC group (*p* = 0.05). These results imply that RA patients may have compensatory activation of immune tolerance.

On the other hand, the AI model was utilized to dissect the contribution of immune cell subsets in separating ICPs belonging to RA patients and the HCs.

### ICPs between RA patients and HCs (for AI model)

The AI model was utilized to evaluate the contribution of immune cell subsets in the separation of ICPs belonging to RA patients and HCs. Three machine learning algorithms - random forest (RF), logistic regression (LR), and support vector machine (SVM), were used to train the AI model using the same dataset.

See **FIGs. 7A-7D**, **8A-8D** and **9A-9D**, and **Table 7.** It was found that the AI model with LR algorithm exhibited superior performance in discriminating ICPs between the RA and HC groups compared to AI models with the RF and SVM algorithms. Hence, the LR algorithm was selected as the core algorithm of the AI model, and its twelve predetermined immune cell subsets **(****FIG. 7D****)** were determined as characterized immune cell subsets, as shown in **Table 8.** The trained AI model can specifically discriminate ICPs between the RA and the HC groups, with no pseudo-positive or pseudo-negative results observed **(****FIGs. 7A-7C****).** Collectively, using the AI model with LR algorithm, ICPs from RA patients can be specifically discriminated from those of HCs.

**Table 7: Model performance- Sensitivity and Specificity of the hold-out set**

| **Algorithm** | **Sensitivity and Specificity of the hold-out set (%)** | |
|---|---|---|
| | HCs (n = 4) | RAs (n = 4) |
| RF | *50.0* | 75.0 |
| LR | 100.0 | 100.0 |
| SVM | *50.0* | 100.0 |

**Table 8: Characterized immune cell subsets of RA disease**

| **Characterized immune cell subsets** | |
|---|---|
| MHC II⁺ monocyte | PD-1⁺CTLA4⁺ CD4 T_{reg} cell |
| CTLA4⁺ CD4 T_{reg} cell | PD-1⁺ PD-L1⁺ CD8 T cell |
| Monocyte | PD-L1⁺ B cell |
| IgM^{hi} in B cell | Memory B cell |
| Eosinophil | MHC II⁺ B cell |
| PD-L1⁺ NK cell | PD-1 in FoxP3⁺ CD4 T_{reg} cell |

### Discussion

In the present example, the flow-based ICP platform was utilized to explore the changes in the ICP in RA patients by comparing the composition of ICP between the RA patients and HCs. It was observed that RA patients exhibited activated ICPs in innate immunity, humoral immunity, and immune tolerance. These changes in ICPs can guide the AI model in discriminating ICPs between RA patients and HCs.

Given that RA disease is an autoimmune disorder that preliminarily affects the joints, understanding immune system changes during its progression can help in the development of diagnostic and treatment tools. Several changes in peripheral ICPs from RA patients were identified, such as increased proportions of CCR2⁺CD4⁺ T cells, peripheral T helper cells, CXCR5⁺CD8⁺ T cells, and CD4⁺CD57⁻ regulatory T cells, as well as a decreased proportion of CD8⁺CD161⁺CD28⁺ T cells. Notably, in the current study, none of the above immune cell subset changes were observed. This discrepancy may be attributed to racial differences from the subject population or finer separation of immune cell lineage in the previous studies. Previous studies typically focused on one or a few immune cell lineages, allowing for more detailed dynamics within those cell lineages. In contrast, the current study aimed for thoroughly characterizing ICPs and identified the obvious change in peripheral ICPs from RA patients, and therefore the current study can provide a comprehensive overview in immune cell changes of RA patients.

In the current study, a significant reduction in eosinophils was identified in RA patients compared to those of the HCs, which is critical for discriminating between ICPs belonging to RA patients and HCs. Eosinophils detect infection or tissue damage, prompting the release of cytokines and cytotoxic proteins to initiate an inflammatory response. Therefore, eosinophils are considered key promoters of inflammation. In studies focusing on the progression of psoriasis, inflammatory bowel diseases, and celiac disease, eosinophilia is commonly observed in affected patients. Given that an increased eosinophil count is positively linked to the onset risk of RA disease, a rise in eosinophils at the onset of RA disease is more plausible. Notably, a controversial role of eosinophils is reported, which interacts with Th2 cells and suppresses the inflammatory response. Eosinophils receive cytokines from Th2 cells (IL-4, IL-13, IL-25), shift M1 macrophages to M2 phenotype, and contribute to the attenuation of inflammation. Considering the significantly elevated levels of IL-4 and IL-13 in both sera and synovial fluid of RA patients as well as the chemotactic effect of IL-4 on eosinophils, the decreased proportion of eosinophils may be attributed to their accumulation in the synovium. Furthermore, the accumulated eosinophils may act as suppressors of inflammatory arthritis rather than promoters. This finding also suggests that the supply of Th2-secreted cytokines could potentially mitigate the progression of RA disease. For instance, IL-25 reduces the activity of Th17-mediated immune response, which greatly contributes to the pathogenesis of RA disease, and suppresses osteoclastogenesis during the onset of RA disease, potentially making it a promising therapy for RA disease.

A decreased proportion of MHC-II⁺ monocytes was observed in RA patients compared to the HCs, and such a decrease contributed significantly to discriminating between ICPs from RA patients and HCs. A previous study suggested an increased proportion of total monocytes in RA patients, whereas the proportional change in the MHC-II⁺ subpopulation remains inconclusive. The current study disclosed that the proportion of MHC-II⁺ monocytes in RA patients decreased. Monocytes express MHC-II after activation, and MHC-II⁺ monocytes participate in the initiation of type 2 immune responses. Considering the elevation of IL-4 and IL-13 levels in both sera and synovial fluid after the initiation of type 2 immune responses, which promote monocyte accumulation in the inflammatory lesion, the decrease in MHC-II⁺ monocytes may be attributed to the chemotaxis of monocytes from circulation to the arthritic lesion. Additionally, IL-4 and IL-13 trigger activated monocytes to differentiate into M2 macrophages, a predominant suppressor of inflammation, which reduces the capacity of Th17 cells and subsequently attenuates the progression of autoimmune disorders, suggesting that MHC-II⁺ monocytes potentially act as suppressors of inflammation in the progression of RA.

### Conclusion

In the present example, the characteristics of ICPs in RA patients and identified 16 immune cell subsets with significantly distinct proportions between RA patients and HCs were thoroughly investigated via classical statistical analysis. Additionally, referring to the results of Tables 6 and 8, seven of these sixteen immune cell subsets also made significant contributions to AI-based ICP discrimination, suggesting that they may play a critical role in the pathogenesis of RA disease. These findings overcame the limitations of previous research on ICP changes in RA patients, which typically focused on only a few aspects of immune system change. Furthermore, the ICP data presented in this study could support the development of novel evaluation tools for early and accurate diagnosis, as well as therapeutic strategies for patients with difficult-to-treat conditions.

In addition, the consistency between classical statistics and AI model in selecting cell populations serves to validate the reliability of the cell populations identified by the AI model. It can be found that the cell populations selected by the AI model show a high degree of overlap with those identified through classical statistics. The AI model selects cell populations with a percentage above classical statistics (p < 0.01) at 75%, indicating that the more significant a cell population is in classical statistics, the higher the probability that the AI model will select it.

### Example 2: Assessing Likelihood of RA Risk in Subject Using Trained AI Model

The whole blood from a subject to be assessed was aliquoted into two in which one was for PBMC isolation and the other was for granulocyte isolation for further immunostaining. See the section "PBMC and WBC Isolation and Immunostaining" described above for details. The difference is that the PBMCs and WBCs obtained the subject were stained via a staining kit, which includes a first pattern, including antibodies against CD3, CD4, CD8, CD14, CD19, CD25, CD45, CD56, CTLA4, FoxP3 and PD-1; a second pattern, including antibodies against CD3, CD4, CD14, CD19, CD45, CD56, HLA-DR, PD-1 and PD-L1; a third pattern, including antibodies against CD3, CD11b, CD14, CD16, CD19, CD45, CD56, CD66b, CD123 and CD193; and a fourth pattern, including antibodies against CD10, CD19, CD21, CD23, CD38, CD45, CD127, IgG and IgM, where the antibodies of each pattern are labeled with fluorescent dyes as shown in **Table 2.** Then, the biomedical data acquisition of the fluorescent intensities of antibodies bound to said 12 characterized immune cell subsets (see **Table 8)** was performed using flow cytometry to obtain a dataset that includes data related to types of the characterized immune cell subsets and proportions thereof.

The trained AI model evaluates the obtained dataset to generate a probability-based risk indicator for the subject based on a predicted probability derived from the LR algorithm. When the predicted probability of a dataset is greater than or equal to the predefined risk threshold value of 0.5, it classifies the subject into a risk group for the RA disease. In this example, the predicted probability of the obtained dataset is 0.89, which exceeds the threshold of 0.5, leading the AI model to classify this subject into a risk group for RA disease. Thus, the subject will be strongly advised to undergo clinical evaluation or monitoring.

Refer to **FIGs. 10A** **and** **10B****.** The SHAP analysis was applied to break down each evaluation into feature contributions. The SHAP force plot offers an intuitive visualization of how individual features influence a single evaluation. The SHAP waterfall plot is another way to visualize individual evaluation, which provides a clearer view of how each feature contributes. The waterfall plot adds each feature's SHAP value sequentially and explicitly shows the stepwise accumulation of features effects. In this representation, red signifies a positive contribution toward the RA disease risk evaluation, while blue indicates a negative contribution. The final evaluation is the sum of all these contributions. Both plots interpret model decisions more transparently by explaining how the model evaluates the contribution of each feature.

The IgM^{hi} in B cell for this dataset is 23.37, which is significantly higher than the average of 8.39 in the HC group and closely aligns with the average of 16.98 observed in the RA disease group. The AI model identifies IgM^{hi} in B cell as the most critical feature contributing to the classification of the present dataset into a risk group for RA disease. The MHC II⁺ monocyte for this dataset is 89.46, which is higher than the average of 76.12 in the RA disease group and closely aligns with the average of 87.93 observed in the HC group. The AI model identifies MHC II⁺ monocyte as the most critical feature contributing to the classification of this dataset into the HC group. As illustrated in **FIGs. 10A** **and** **10B****,** within the feature contribution analysis of this dataset, the positive contributions (represented in red) exceed the negative contributions (represented in blue). Consequently, the AI model aggregates these contributions and classifies this subject into a risk group for RA disease.

In summary, the present invention not only can use to assess the likelihood of RA disease risk in a subject, but also can provide prediction about immunotherapy based on states of PD-L1, PD-1 and T cells in the 12 immune cell subsets selected by said AI model. For example, a subject can be administrated with Atezolizumab for treatment while an amount of PD-L1 expression of immune cells increases; can be administrated with Nivolumab or Pembrolizumab for treatment while an amount of PD-1 expression of immune cells increases; or can be supplemented with immune cells such as NK, DC, Cytokine-induced Killer (CIK), and T cells for treatment while T cells decreases.

Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein.

All references cited herein are incorporated herein by reference to the full extent allowed by law. The discussion of those references is intended merely to summarize the assertions made by their authors. No admission is made that any reference (or a portion of any reference) is relevant prior art. Applicants reserve the right to challenge the accuracy and pertinence of any cited reference.

## Claims

1. A method of processing biomedical data to generate a probability-based risk indicator for assessing the likelihood of a rheumatoid arthritis (RA) disease risk in a subject, comprising steps of:
(a) staining peripheral blood mononuclear cells (PBMCs) and/or white blood cells (WBCs) from the subject by using a staining kit, wherein the staining kit comprises:
a first pattern, comprising antibodies against CD3, CD4, CD8, CD14, CD19, CD25, CD45, CD56, CTLA4, FoxP3 and PD-1;
a second pattern, comprising antibodies against CD3, CD4, CD14, CD19, CD45, CD56, HLA-DR, PD-1 and PD-L1;
a third pattern, comprising antibodies against CD3, CD11b, CD14, CD16, CD19, CD45, CD56, CD66b, CD123 and CD193; and
a fourth pattern, comprising antibodies against CD10, CD19, CD21, CD23, CD38, CD45, CD127, IgG and IgM,
wherein the antibodies of each pattern are labeled with fluorescent dyes;
(b) performing biomedical data acquisition of fluorescent intensity of each antibody bound to characterized immune cell subsets of an RA disease by using flow cytometry to obtain a dataset comprising data related to types of the characterized immune cell subsets and proportions thereof; and
(c) evaluating the dataset by using an artificial intelligent (AI) model to generate the probability-based risk indicator for the subject, thereby stratifying the subject into a risk category for the RA disease, wherein the generated probability-based risk indicator reflects the likelihood of the subject being at risk for the RA disease, providing a basis for further clinical evaluation or monitoring.

2. The method of claim 1, wherein the use of the AI model comprises applying a machine learning algorithm based on a logistic regression (LR) algorithm.

3. The method of claims 1 or 2, wherein the step (c) of evaluating the dataset comprises the following steps performed by the AI model:
generating the probability-based risk indicator based on a predicted probability derived from the machine learning algorithm utilizing the LR algorithm, and
outputting the probability-based risk indicator as an assessment result that classifies the subject into a risk group for the RA disease when the predicted probability meets or exceeds a predefined risk threshold value.

4. The method of any one of claims 1-3, wherein the characterized immune cell subsets of the RA disease comprises eosinophil, memory B cell, IgM^{hi} in B cell, MHC II⁺ B cell, monocyte, MHC II⁺ monocyte, PD-L1⁺ NK cell, PD-L1⁺ B cell, PD-1⁺ PD-L1⁺ CD8 T cell, CTLA4⁺ CD4 T_{reg} cell, PD-1⁺ FoxP3⁺ CD4 T_{reg} cell, and PD-1⁺ CTLA4⁺ CD4 T_{reg} cell.

5. The method of any one of claims 1-4, wherein the characterized immune cell subsets of the RA disease are identified by steps of:
(i) staining the PBMCs and/or the WBCs of a plurality of healthy controls and a plurality of patients having the RA disease, respectively, by using a second staining kit;
(ii) performing data acquisition of fluorescent intensity of each antibody bound to the PBMCs and/or the WBCs of the healthy controls and the patients, respectively, by using flow cytometry;
(iii) identifying immune cell subsets in the PBMCs and/or the WBCs of the healthy controls and the patients, respectively, by using a pedigree method to obtain a dataset comprising data related to types of the immune cell subsets and proportions thereof;
(iv) performing data preprocessing of the dataset; and
(v) evaluating the preprocessed dataset by using the AI model to obtain immune cell subsets of the patients distinguishable from those of the healthy controls as the characterized immune cell subsets of the RA disease.

6. The method of claim 5, wherein the step (v) of evaluating the preprocessed dataset comprises the following steps performed by the AI model:
performing a feature selection from the preprocessed data by using a Boruta algorithm to obtain predetermined immune cell subsets of the RA disease; and
applying data of the predetermined immune cell subsets to train the AI model with a machine learning algorithm selected from at least one of a random forest (RF) algorithm, a logistic regression (LR) algorithm and a support vector machines (SVM) algorithm so as to determine the characterized immune cell subsets.

7. A staining kit, comprising a first pattern comprising antibodies against CD3, CD4, CD8, CD14, CD19, CD25, CD45, CD56, CTLA4, FoxP3 and PD-1; a second pattern comprising antibodies against CD3, CD4, CD14, CD19, CD45, CD56, HLA-DR, PD-1 and PD-L1; a third pattern comprising antibodies against CD3, CD11b, CD14, CD16, CD19, CD45, CD56, CD66b, CD123 and CD193; and a fourth pattern comprising antibodies against CD10, CD19, CD21, CD23, CD38, CD45, CD127, IgG and IgM, wherein the antibodies of each pattern are labeled with fluorescent dyes.
